# EUROPEAN PATENT APPLICATION

(11) **EP 4 410 220 A1**
(43) Date of publication of application: **07.08.2024**
(21) Application number: 22898766.5
(22) Date of filing: 17.08.2022
(51) Int. Cl.: A61B 10/00, A61B 5/01, A61B 5/00, A61B 5/024, A61B 5/11, G16H 20/00

(54) **WEARABLE DEVICE AND METHOD FOR PROVIDING SERVICE ON BASIS OF USER BODY TEMPERATURE**

(30) Priority: 29.11.2021 KR 20210167797; 04.02.2022 KR 20220014855
(71) Applicant: Samsung Electronics Co., Ltd., Suwon-si, Gyeonggi-do 16677 (KR)
(72) Inventor: KWON, Hyoujoo, Suwon-si Gyeonggi-do 16677 (KR); KIM, Seungju, Suwon-si Gyeonggi-do 16677 (KR); JE, Seongmin, Suwon-si Gyeonggi-do 16677 (KR)
(74) Representative: Gulde & Partner
(86) International application number: PCT/KR2022/012261
(87) International publication number: WO 2023/096082

(57) **Abstract**

According to an embodiment, a wearable device comprises a non-contact temperature sensor, a biometric sensor, a motion sensor, memory, and at least one processor. The at least one processor may be set to: acquire first body temperature data of a user according to a first period, while determining that the user is in a stable condition within a first cycle; use the motion sensor to acquire values, for indicating changes in the motion of the user, according to a second period, while determining that the user is in an unstable condition within the first cycle; acquire second body temperature data in response to determining that each of the values for indicating changes in the motion of the user is smaller than a reference value; acquire first information about the trend of the change in the user body temperature within the first cycle; and provide a service at least partly on the basis of the first information.

## Description

### [Technical Field]

The following descriptions relate to wearable devices and methods for providing services based on the user's body temperature.

### [Background Art]

Various women's health services can be provided through wearable devices. The women's health service may refer to a service that helps in pregnancy-planning or birth control by providing a woman's (or user)'s menstrual cycle, a fertile window (or ovulation date), and/or a contraceptive period. Representatively, women's health services based on the standard days method (SDM) through the body temperature of the user may be provided through a wearable device.

### [Disclosure]

### [Technical Problem]

To identify a menstrual cycle, a basal body temperature may be used. In order to use the basal body temperature method, a wearable device needs to identify a basal body temperature value of the user (or woman). In a case that a contact temperature sensor is used, when the thermal equilibrium state is not reached, there is a problem that the exact temperature is not measured. When the exact temperature is not measured, it is difficult to provide women's health services.

The technical problems to be achieved in this document are not limited to those described above, and other technical problems not mentioned herein will be clearly understood by those having ordinary knowledge in the art to which the present disclosure belongs, from the following description.

### [Technical Solution]

According to various embodiments, an wearable device may comprise a non-contact temperature sensor disposed on position distant from a part of body of a user wearing the wearable device, and measuring a temperature based on infrared light, a biometric sensor, a motion sensor, a memory, and an at least one processor operably coupled to the non-contact temperature sensor, the biometric sensor, the motion sensor, and the memory, configured to obtain, while identifying that a user is in a stable state within a first cycle, using the non-contact temperature sensor, first body temperature data of the user according to a first period; while identifying that the user is in an unstable state within the first period, obtain, using the motion sensor, values for indicating a change of motion of the user according to a second period distinct from the first period; in response to identifying that each of the values for indicating the change of motion of the user is less than a reference value, obtain second body temperature data through the non-contact temperature sensor; obtain, based on the first body temperature data and the second body temperature data, first information on a trend in which body temperature of the user changes within the first cycle, and provide a service based at least part on the first information.

According to various embodiments, a method of a wearable device may comprise obtaining, while identifying that a user is in a stable state within a first cycle, using a non-contact temperature sensor, first body temperature data of the user according to a first period; while identifying that the user is in an unstable state within the first cycle, obtaining, using a motion sensor, values for indicating a change of motion of the user according to a second period distinct from the first period; in response to identifying that each of the values for indicating the change of motion of the user is less than a reference value, obtaining second body temperature data through the non-contact temperature sensor; obtaining, based on the first body temperature data and the second body temperature data, first information on a trend in which body temperature of the user changes within the first cycle; and providing a service based at least part on the first information.

According to various embodiments, a non-transitory computer readable storage medium may store one or more programs, the one or more programs including instructions, which, when being executed by at least one processor of a wearable device with a non-contact temperature sensor, a biometric sensor, a motion sensor, and a memory, cause the electronic device to obtain, while identifying that a user is in a stable state within a first cycle, using the non-contact temperature sensor, first body temperature data of the user according to a first period; while identifying that the user is in an unstable state within the first cycle, obtain, using the motion sensor, values for indicating a change of motion of the user according to a second period distinct from the first period; in response to identifying that each of the values for indicating the change of motion of the user is less than a reference value, obtain second body temperature data through the temperature sensor; obtain, based on the first body temperature data and the second body temperature data, first information on a trend in which body temperature of the user changes within the first cycle; and provide a service based at least part on the first information.

### [Advantageous Effects]

According to an embodiment, a wearable device can identify a body temperature of a user by using a non-contact temperature sensor. The wearable device can pattern a change in body temperature within a first cycle (e.g., one day). The wearable device can also pattern a change in body temperature within a second cycle (e.g., one month or menstrual cycle). The wearable device can identify a change in a user's body temperature by patterning a change in body temperature in the first cycle and a change in body temperature in the second cycle. The wearable device can perform an operation related to woman's health services based on a change in the user's body temperature.

The effects that can be obtained from the present disclosure are not limited to those described above, and any other effects not mentioned herein will be clearly understood by those having ordinary knowledge in the art to which the present disclosure belongs, from the following description.

### [Description of the Drawings]

FIG. 1 is a block diagram of an electronic device in a network environment according to various embodiments.
FIGS. 2A and 2B are perspective views of an electronic device according to an embodiment.
FIG. 3 is an exploded perspective view of an electronic device according to an embodiment.
FIG. 4 is a simplified block diagram of a wearable device according to an embodiment.
FIG. 5 is a specific example of a sensor of a wearable device according to an embodiment.
FIGS. 6A and 6B are specific examples of a non-contact type IR sensor of a wearable device according to an embodiment.
FIG. 7 is a diagram illustrating an example of an operation of a wearable device according to an embodiment.
FIG. 8 is a flowchart illustrating an operation of a wearable device according to an embodiment.
FIG. 9 is another flowchart illustrating an operation of a wearable device according to an embodiment.
FIG. 10 is a diagram illustrating an example of an operation of a wearable device according to an embodiment.
FIG. 11 is a diagram illustrating another example of an operation of a wearable device according to an embodiment.
FIG. 12 is a diagram illustrating another example of an operation of a wearable device according to an embodiment.
FIG. 13 is a diagram illustrating another example of an operation of a wearable device according to an embodiment.
FIG. 14 is a diagram illustrating another example of an operation of a wearable device according to an embodiment.
FIG. 15 is a diagram illustrating another example of an operation of a wearable device according to an embodiment.
FIG. 16 is another flowchart illustrating an operation of a wearable device according to an embodiment.
FIG. 17 is a diagram illustrating another example of an operation of a wearable device according to an embodiment.
FIG. 18 is a diagram illustrating another example of an operation of a wearable device according to an embodiment.
FIG. 19 is a flowchart illustrating an operation of a wearable device according to an embodiment.
FIG. 20 is a diagram illustrating another example of an operation of a wearable device according to an embodiment.
FIG. 21 is a flowchart illustrating an operation of a wearable device according to an embodiment.
FIG. 22 is a diagram illustrating another example of an operation of a wearable device according to an embodiment.
FIG. 23 is a flowchart illustrating an operation of a wearable device according to an embodiment.
FIG. 24 is a diagram illustrating another example of an operation of a wearable device according to an embodiment.

### [Mode for Invention]

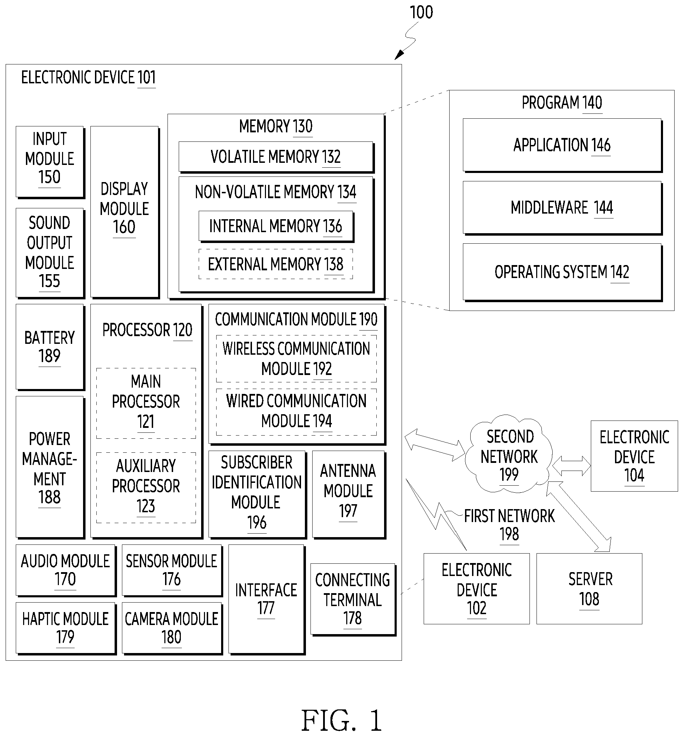

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

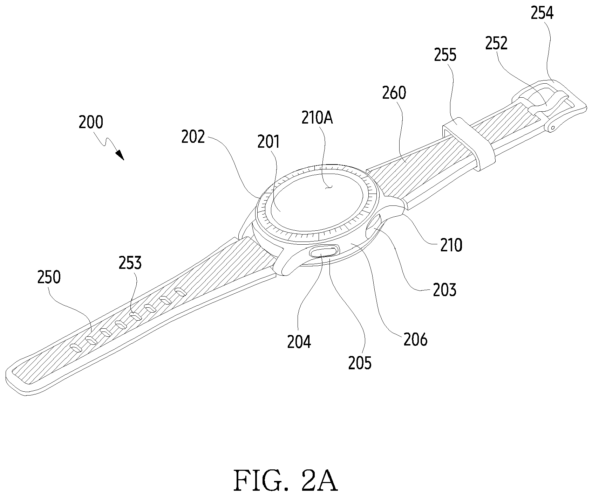

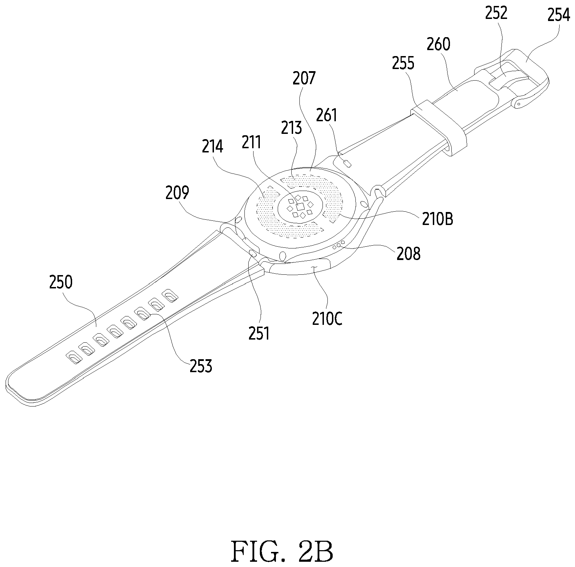

FIGS. 2A and 2B are perspective views of an electronic device according to an embodiment.

Referring to FIGS. 2A and 2B, according to an embodiment, an electronic device 200 (e.g., the electronic device 101 of FIG. 1) may include a housing 210A including a first surface (or a front surface) 210A, a second surface (or a rear surface) 210B, and a side surface 210C surrounding a space between the first surface 210A and the second surface 210B, and binding members 250 and 260 connected to at least a part of the housing 210 and detachably couple the electronic device 200 to a part of a user's body (e.g., a wrist, an ankle, etc.). In another embodiment (not illustrated), the housing may refer to a structure forming some of the first surface 210A, the second surface 210B, and the side surface 210C of FIGS. 2A and 2B. According to an embodiment, at least a part of the first surface 210A may be formed by a substantially transparent front plate 201 (e.g., a glass plate including various coating layers, or a polymer plate). The second surface 210B may be formed by a substantially opaque rear plate 207. For example, the rear plate 207 may be formed by coating or colored glass, ceramic, polymer, metal (e.g. aluminum, stainless steel (STS), or magnesium), or a combination of at least two of the materials. The side surface 210C may be formed by a side bezel structure (or "side member") 206 coupled to the front plate 201 and the rear plate 207 and including a metal and/or a polymer. In some embodiments, the rear plate 207 and the side bezel structure 206 may be integrally formed and may include the same material (e.g., a metal material such as aluminum). The binding members 250 and 260 may be formed of various materials and shapes. An integral unit link and a plurality of unit links may be formed to flow with each other by a woven fabric, leather, rubber, urethane, metal, ceramic, or a combination of at least two of the materials.

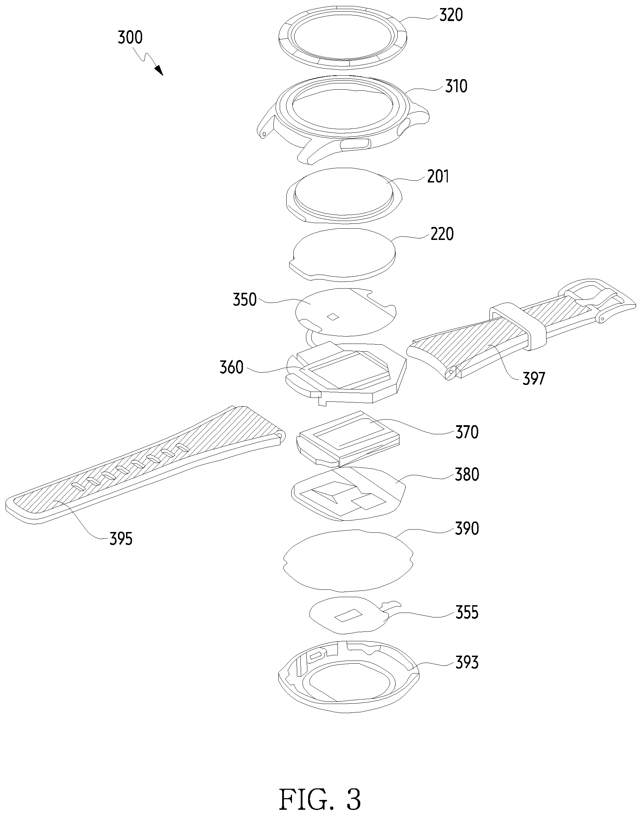

According to an embodiment, the electronic device 200 may include at least one of a display 220 (refer to FIG. 3), audio modules 205 and 208, a sensor module 211, key input devices 202, 203 and 204, and a connector hole 209. In some embodiments, the electronic device 200 may omit at least one (e.g., key the input devices 202, 203 and 204, the connector hole 209, or the sensor module 211) of the components or may additionally include another component.

The display 220 may be visually exposed, for example, through a substantial part of the front plate 201. The shape of the display 220 may be a shape corresponding to the shape of the front plate 201, and may have various shapes such as a circle, an ellipse, or a polygon. The display 220 may be coupled to, or disposed adjacent to, a touch detecting circuit, a pressure sensor capable of measuring the intensity (pressure) of a touch, and/or a fingerprint sensor.

The audio modules 205 and 208 may include a microphone hole 205 and a speaker hole 208. In the microphone hole 205, a microphone for obtaining an external sound may be disposed inside, and in some embodiments, a plurality of microphones may be disposed to detect the direction of the sound. The speaker hole 208 may be used as an external speaker and a receiver for calls. In some embodiments, the speaker hole 208 and the microphone hole 205 may be implemented as one hole, or a speaker may be included without the speaker hole 208 (e.g., a piezo speaker).

The sensor module 211 may generate an electrical signal or data value corresponding to an internal operating state of the electronic device 200 or an external environmental state. The sensor module 211 may include, for example, a biometric sensor module 211 (e.g., an HRM sensor) disposed on the second surface 210B of the housing 210. The electronic device 200 may further include at least one of a sensor module not illustrated, for example, a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illumination sensor.

The sensor module 211 may include electrode regions 213 and 214 forming a part of the surface of the electronic device 200 and a bio-signal detection circuit (not illustrated) electrically connected to the electrode regions 213 and 214. For example, the electrode regions 213 and 214 may include a first electrode region 213 and a second electrode region 214 disposed on the second surface 210B of the housing 210. The sensor module 211 may be configured such that the electrode regions 213 and 214 obtain an electrical signal from a part of the user's body, and a bio-signal detection circuit detects bio-information of the user based on the electrical signal.

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key may have a shape corresponding to the shape of the front plate 201. In another embodiment, the electronic device 200 may not include some or all of the above-described key input devices 202, 203, and 204, and the not included key input devices 202, 203, and 204 may be implemented in other forms such as a soft key on the display 220. The connector hole 209 may accommodate connectors (e.g., USB connectors) for transmitting and receiving power and/or data to and from external electronic devices, and include another connector hole (not illustrated) capable of accommodating a connector for transmitting and receiving audio signals to and from an external electronic device. For example, the electronic device 200 may further include a connector cover (not illustrated) that covers at least a part of the connector hole 209 and blocks the inflow of external foreign materials into the connector hole.

The binding members 250 and 260 may be detachably attached to at least a part of the housing 210 using locking members 251 and 261. The binding members 250 and 260 may include one or more of a fixing member 252, a fixing member fastening hole 253, a band guide member 254, and a band fixing ring 255.

The fixing member 252 may be configured to fix the housing 210 and the binding members 250 and 260 to a part of the user's body (e.g., a wrist, an ankle, etc.). Corresponding to the fixing member 252, the fixing member fastening hole 253 may fix the housing 210 and the binding members 250 and 260 to a part of the user's body. The band guide member 254 may be configured to limit a movement range of the fixing member 252 when the fixing member 252 is fastened to the fixing member fastening hole 253, and thus the binding members 250 and 260 may be closely coupled to a part of the user's body. In a state in which the fixing member 252 and the fixing member fastening hole 253 are fastened, the band fixing ring 255 may limit the movement range of the binding members 250 and 260.

FIG. 3 is an exploded perspective view of an electronic device according to an embodiment.

Referring to FIG. 3, the electronic device 300 (e.g., the electronic device 101 of FIG. 1 or the electronic device 200 of FIGS. 2A to 2B) may include a side bezel structure 310, a wheel key 320, a front plate 201, a display 220, a first antenna 350, a second antenna 355, and a support member 360(e.g., a bracket), a battery 370, a printed circuit board 380, a sealing member 390, a rear plate 393, and binding members 395 and 397. At least one of the components of the electronic device 300 may be the same as, or similar to, at least one of the components of the electronic device 200 of FIGS. 1 and 2A to 2B, and a repeated description thereof will be omitted. The support member 360 may be disposed inside the electronic device 300 to be connected to the side bezel structure 310 or may be integrally formed with the side bezel structure 310. The support member 360 may be formed of, for example, a metal material and/or a non-metal (e.g., a polymer) material. In the support member 360, the display 220 may be coupled to one surface and the printed circuit board 380 may be coupled to the other surface. A processor, a memory, and/or an interface may be mounted on the printed circuit board 380. The processor may include, for example, one or more of a central processing unit, a graphic processing unit (GPU), an application processor, a sensor processor, or a communication processor.

The memory may include, for example, a volatile memory or a nonvolatile memory. The interface may include, for example, a high definition multimedia interface (HDMI), a universal serial bus (USB) interface, an SD card interface, and/or an audio interface. For example, the interface may electrically or physically connect the electronic device 300 to an external electronic device, and may include a USB connector, an SD card/MMC connector, or an audio connector.

The battery 370 is a device for supplying power to at least one component of the electronic device 300, and may include, for example, a non-rechargeable primary battery, a rechargeable secondary battery, or a fuel battery. At least a part of the battery 370 may be disposed on substantially the same plane as, for example, the printed circuit board 380. The battery 370 may be integrally disposed inside the electronic device 200 or may be detachably disposed from the electronic device 200.

The first antenna 350 may be disposed between the display 220 and the support member 360. The first antenna 350 may include, for example, a near field communication (NFC) antenna, a wireless charging antenna, and/or a magnetic secure transmission (MST) antenna. For example, the first antenna 350 may perform short-range communication with an external device, wirelessly transmit and receive power required for charging, and may transmit a short-range communication signal or a self-based signal including payment data. In another embodiment, an antenna structure may be formed by the side bezel structure 310 and/or a part of the support member 360 or a combination thereof.

The second antenna 355 may be disposed between the printed circuit board 380 and the rear plate 393. For example, the second antenna 355 may include a near field communication (NFC) antenna, a wireless charging antenna, and/or a magnetic secure transmission (MST) antenna. For example, the second antenna 355 may perform short-range communication with an external device, wirelessly transmit and receive power required for charging, and may transmit a short-range communication signal or a self-based signal including payment data. In another embodiment, an antenna structure may be formed by the side bezel structure 310 and/or a part of the rear plate 393 or a combination thereof.

The sealing member 390 may be positioned between the side bezel structure 310 and the rear plate 393. The sealing member 390 may be configured to block moisture and foreign substances from flowing into the space surrounded by the side bezel structure 310 and the rear plate 393 from the outside.

According to an embodiment, the wearable device (e.g., the electronic device 200 illustrated in FIGS. 2A and 2B ) may be worn by a user (or a woman) to operate. The wearable device may provide woman health services by identifying (or obtaining) the user's body temperature. The wearable device may be used to provide a woman health service.

For example, the women's health service may include a service that provides a woman(or user)'s menstrual cycle, a fertile window (or ovulation date), and/or contraceptive period. The menstrual cycle of a woman may be determined according to the ovulation date. The menstrual date is determined after about 14 days (or on average 14 days) based on the ovulation date. The fertile window refers about five days before and after the ovulation day. The contraceptive period is determined by a non- fertile window.

The wearable device may provide information on the fertile window or contraceptive period to the user by identifying the ovulation date of the user (or woman). As a method for identifying the ovulation date, symptom contraception and/or date contraception may be used. Symptom contraception includes basal body temperature (BBT) and urine tests (e.g., luteinizing hormone test, LH test). Date contraception includes the rhythm method and the standard days method.

Date contraception is a statistical contraceptive method, which is less accurate than symptom contraception. In addition, the urine test method among Symptom contraception has the disadvantage of requiring a test device. Accordingly, the basal body temperature method may be used to provide women's health services through the wearable device.

For example, the wearable device may identify (or obtain) a user's body temperature value within the first cycle (e.g., 24 hours or one day). The wearable device may identify a trend in which the body temperature of the user changes within the first cycle based on the body temperature values of the user obtained according to the first cycle. The wearable device may identify a trend in which the body temperature of the user changes within the second cycle (e.g., 30 days or one month). The wearable device may provide a woman health service based on a trend in which the body temperature changes within the second cycle.

An operation of the wearable device according to the above-described embodiment may be described below. The wearable device described below may correspond to the electronic device 101 of FIG. 1 and/or the electronic device 200 of FIGS. 2A and 2B. The wearable electronic device may be implemented in various forms that may be worn to a user, such as a smart watch, a smart band, a smart ring, a wireless earphone, or a smart glass.

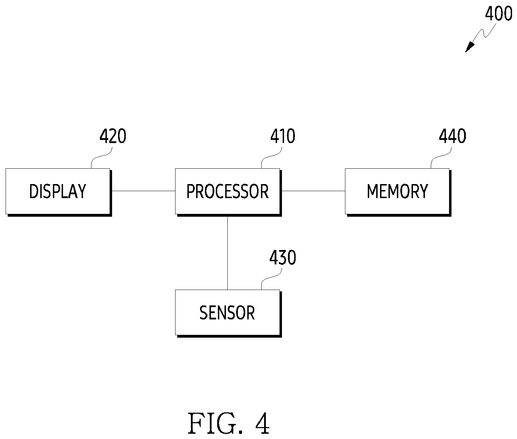

FIG. 4 is a simplified block diagram of a wearable device according to an embodiment.

Referring to FIG. 4, a wearable device 400 may correspond to the electronic device 101 of FIG. 1 and/or the electronic device 200 of FIGS. 2A and 2B. The wearable device 400 may include a processor 410, a display 420, a sensor 430, and/or a memory 440. According to an embodiment, the wearable device 400 may include at least one of the processor 410, the display 420, the sensor 430, and the memory 440. For example, at least a part of the processor 410, the display 420, the sensor 430, and the memory 440 may be omitted according to an embodiment.

According to an embodiment, the processor 410 may correspond to the processor 120 of FIG. 1. The processor 410 may be operatively coupled with or connected with the display 420, the sensor 430, and the memory 440. For example, the processor 410 may control the display 420, the sensor 430, and the memory 440. The display 6420, the sensor 430, and the memory 440 may be controlled by the processor 410. For example, the processor 120 may be configured with at least one processor. The processor 120 may include at least one processor.

According to an embodiment, the processor 410 may include a hardware component for processing data based on one or more instructions. The hardware components for processing data may include, for example, an arithmetic and logic unit (ALU), a field programmable gate array (FPGA), and/or a central processing unit (CPU).

According to an embodiment, the processor 410 may determine an operation time point of the sensor 430. The processor 410 may control the operation of the sensor 430. The processor 410 may process information obtained from the sensor 430.

For example, the processor 410 may obtain information on a trend in which a user's body temperature changes within a first cycle (e.g., 24 hours or one day). For example, the processor 410 may pattern a circadian rhythm of a user using a pattern algorithm based on body data(e.g., body temperature data, skin temperature data, heart rate (HR) data, heart rate variability (HRV) data, activity time data, or sleep time data) obtained from the sensor 430 for one day. A detailed operation of the processor 410 for patterning the circadian rhythm of the user using the pattern algorithm will be described later.

For example, the processor 410 may obtain information on a trend in which a user's body temperature changes within a second cycle (e.g., one month or menstrual cycle). For example, the processor 410 may identify a basal body temperature (BBT) based on the circadian rhythm pattern of the user. Based on the basal body temperature patterning algorithm, the user's basal body temperature may be patterned within the second cycle. A detailed operation of the processor 410 for patterning the user's basal body temperature within the second cycle will be described later.

For example, the processor 410 may identify (or predict or estimate) a fertile window and contraceptive period based on information on the trend in which the user's body temperature changes within the second cycle. The processor 410 may identify the fertile window or the contraceptive period based on the user's basal body temperature pattern within the second cycle.

According to an embodiment, the wearable device 400 may include the display 420. The display 420 may be used to display various screens. For example, the display 420 may be used to output content, data, or signal through screens. For example, the display 420 may display a screen processed by the processor 410. For example, the display 420 may be used to display a guide for the time point in which the event related to the user will be occurred. For example, the display 420 may correspond to the display module 160 of FIG. 1.

According to an embodiment, the wearable device 400 may include the sensor 430. The sensor 430 may be used to obtain various external information. For example, the sensor 430 may be used to obtain data on the user's body. For example, the sensor 430 may be used to obtain a user's body temperature data, heart rate data, and/or motion data. For example, the sensor 430 may be configured with at least one sensor. The sensor 430 may include at least one sensor. For example, the sensor 430 may correspond to the sensor module 176 of FIG. 1.

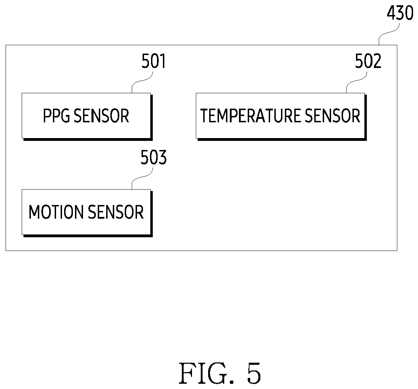

For example, the sensor 430 may include at least one of a photoplethysmography (PPG) sensor, a temperature sensor (or body temperature sensor), and a motion sensor. A detailed example of the sensor 430 including the PPG sensor, the temperature sensor, and the motion sensor will be described later in FIG. 5.

According to an embodiment, the wearable device 400 may include the memory 440. The memory 440 may be used to store information or data. For example, the memory 440 may be used to store data obtained from a user. For example, the memory 440 may correspond to the memory 130 of FIG. 1. For example, the memory 440 may be a volatile memory unit or units. For example, the memory 440 may be a nonvolatile memory unit or units. For another example, the memory 440 may be another type of computer-readable medium, such as a magnetic or optical disk. For example, the memory 440 may store data obtained based on an operation (e.g., an algorithm execution operation) performed by the processor 410. For example, the memory 440 may store data (e.g., body temperature data) obtained by the sensor 430.

Although not illustrated, the wearable device 400 may further include a communication circuit. The communication circuit 320 may correspond to at least a part of the communication module 190 of FIG. 1. For example, the communication circuitry may be used for various radio access technology (RAT). For example, the communication circuit may be used to perform Bluetooth communication or wireless local area network (WLAN) communication. As another example, the communication circuitry may be used to perform cellular communication. For example, the processor 410 may establish a connection with an external electronic device through the communication circuit. As another example, the processor 410 may establish a connection with the server through the communication circuit.

FIG. 5 is a specific example of a sensor of a wearable device according to an embodiment.

Referring to FIG. 5, the sensor 430 may include a sensor for obtaining biometric data of a user. The sensor 430 may include a biometric sensor. The sensor 430 may be used to identify (or detect) at least one of blood pressure, electrocardiogram, heart rate variability (HRV), heart rate monitor (HRM), photoplethysmography (PPG), sleep interval, skin temperature, heart rate, blood flow, blood sugar, oxygen saturation, pulse wave, and electrocardiogram (ECG). For example, the processor 410 may obtain a waveform of the bio-signal based on the PPG or ECG through the sensor 430. For example, the bio-signal may include a photoplethysmography, a pulse wave, or an electrocardiogram. The processor 410 may identify at least one of blood pressure, HRV, HRM, skin temperature, blood flow, blood sugar, and oxygen saturation based on the waveform of the bio-signal.

For example, the processor 410 may obtain information on dispersion or deviation of inter-beat interval (IBI) information between peak-to-peak of a waveform based on the photoplethysmography information. The processor 410 may obtain information on the regularity or variability of the heart rate based on information on the distribution of IBI information or information on the deviation of IBI information. As another example, the processor 410 may obtain information on the regularity or variability of a heart rate based on frequency analysis of the heart rate signal.

According to an embodiment, the sensor 430 may include a PPG sensor 501, a temperature sensor 502, and/or a motion sensor 503.

For example, the PPG sensor 501 may be used to measure a pulse (or a change in the amount of blood in a blood vessel) by identifying a change in the amount of light sensitivity according to a change in the volume of a blood vessel. The processor 410 may identify a sleep state or a non-sleep state (or an activity state) of the user based on the obtained biometric data through the PPG sensor 501. For example, the PPG sensor 501 may include one or more photodiodes (PDs) and one or more light emitting diodes (LEDs).

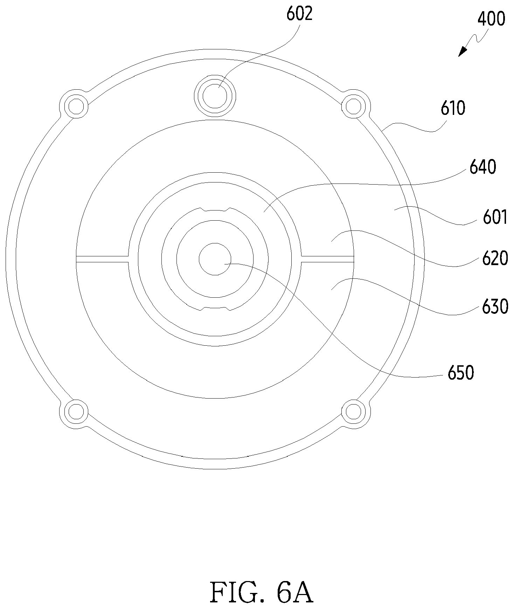

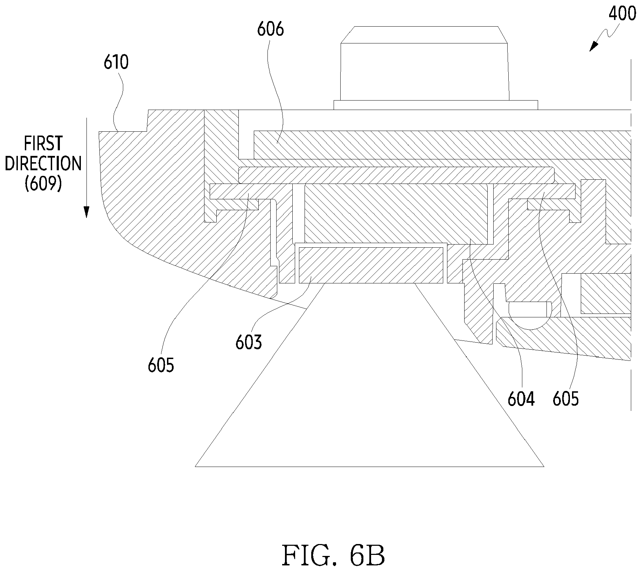

For example, the temperature sensor 502 may be used to identify a user's body temperature. The temperature sensor 502 may include a non-contact infrared radiation (IR) temperature sensor or a contact temperature sensor. For example, the processor 410 may measure the temperature in a state where the contact type temperature sensor contacts a part of the user's body. As another example, the processor 410 may measure the temperature based on infrared light through a non-contact IR temperature sensor disposed to be spaced apart from a part of the user's body (e.g., wrist). A structure in which the non-contact IR temperature sensor is included in the wearable device 400 may be described with reference to FIGS. 6A and 6B.

According to an embodiment, the processor 410 may identify (or measure) the temperature in a part (e.g., wrist) of the user's body through the temperature sensor 502. the temperature measured in a part of the user's body may be distinct from the temperature measured in another part of the user's body (e.g., mouth, forehead, or armpit). The processor 410 may correct a temperature measured in a part (e.g., wrist) of the user to a temperature identified in another part of the user's body. For example, the temperature identified in a part of the user's body may be identified to be lower than the temperature identified in another part of the user's body by a designated temperature value. The processor 410 may correct the temperature measured in a part of the user's body to a temperature identified in another part of the user's body by adding a designated temperature value to the temperature measured in a part of the user's body.

For example, the motion sensor 503 may be used to obtain data (e.g., a value for motion) about the motion of the wearable device 400 (or a user). For example, the motion sensor 503 may include an acceleration sensor, a gyro sensor, a geomagnetic sensor, or an atmospheric pressure sensor. The acceleration sensor may identify(or measure) and detect the acceleration of the wearable device 400 in three directions of the x-axis, the y-axis, and the z-axis. The gyro sensor may identify (or measure, detect) the angular velocity of the wearable device 400 in three directions of the x-axis, the y-axis, and the z-axis. The geomagnetic sensor may identify (or measure, detect) a value for bearing by identifying geomagnetism. The atmospheric pressure sensor may identify (or measure, detect) atmospheric pressure around the wearable device 400.

Although not illustrated, the sensor 430 may further include various sensors for obtaining (or identifying, measuring, and detecting) various biometric data of a user.

For example, the sensor 430 may include an HRV sensor. The processor 410 may measure the regularity or variability of the heart rate through an HRV sensor. The processor 410 may obtain information on the regularity or variability of the heart rate through the HRV sensor.

For example, the sensor 430 may include an electrode sensor. The processor 410 may identify (or measure) electrodermal activity (EDA) through the electrode sensor. The processor 410 may identify information on the skin tension based on the EDA.

For example, the sensor 430 may include a blood sugar sensor. The processor 410 may identify a user's blood sugar level by identifying (or measuring) a current generated by causing an electro-chemical reaction with blood sugar in blood.

FIGS. 6A and 6B are specific examples of a non-contact type IR sensor of a wearable device according to an embodiment.

Referring to FIG. 6A , the first surface 601 of the housing 610 of the wearable device 400 may include a region 602 through which radiated electromagnetic waves are transmitted. For example, the region 602 may be located in a partial region of the first surface 601 of the housing 610. For example, the first surface 601 of the housing 610 may correspond to the second surface 210B of the housing 210 as illustrated in FIGS. 2A and 2B. Although not illustrated, the first surface 601 of the housing 610 may include at least one region for identifying biometric data of a user, including the region 602 through which electromagnetic waves are transmitted.

According to an embodiment, the first surface 601 of the housing 610 of the wearable device 400 may include a first electrode 620 and a second electrode 630. The first electrode 620 may correspond to the first electrode region 213 of FIG. 2B. The second electrode 630 may correspond to the second electrode region 214 of FIG. 2B.

According to an embodiment, the first surface 601 of the housing 610 of the wearable device 400 may include a PD 640 and an LED 650 of the PPG sensor.

Referring to FIG. 6B, the wearable device 400 may include a housing 610, a lens 603, a non-contact IR temperature sensor 604, an adhesive part 605, and a printed circuit board(PCB) 606. The PCB 606 may be disposed within the housing 610. The non-contact IR temperature sensor 604 may be disposed on one surface of the PCB 606 facing the first direction 609. At least one component may be disposed between the non-contact IR temperature sensor 604 and the PCB 606. For example, a flexible printed circuit board (FPCB) (not illustrated) may be disposed on one surface of the PCB 606 facing the first direction 609. The FPCB may be connected to one surface of the PCB 606 facing the first direction 609. The non-contact IR temperature sensor 604 may be disposed on one surface of the FPCB facing the first direction 609. The non-contact IR temperature sensor 604 may be connected to one surface of the FPCB facing the first direction 609. The lens 603 may be disposed on one surface of the IR temperature sensor 604 facing the first direction. The lens 603 may be disposed toward the rear glass.

Referring to FIGS. 6A and 6B, the IR temperature sensor 604 may be used to identify electromagnetic waves (e.g., 3µm to 5µm band of MWIR (medium wave infra-red) and 8µm to 14µm band of long wave infra-red (LWIR)) emitted from external objects (e.g., the user's skin). For example, the higher the temperature of the external object, the shorter the wavelength of the electromagnetic wave emitted from the external object, and the amount of radiation energy may increase. The non-contact IR temperature sensor 604 may include a thermopile. The thermopile may include a hot junction and a cold junction. The non-contact IR temperature sensor 604 may identify the temperature by using the Seeback effect generated in proportion to the magnitude of the temperature difference between the hot junction and the cold junction.

According to an embodiment, the non-contact IR temperature sensor 604 may measure the temperature faster than the contact temperature sensor. When the contact temperature sensor is used, the processor 410 may identify (or measure) the temperature after a thermal equilibrium state with the skin temperature is achieved. When the contactless IR temperature sensor 604 is used, the processor 410 may identify (or measure) the temperature of the skin in a shorter time. When the non-contact IR temperature sensor 604 is used, the processor 410 may identify (or measure) the temperature of the skin even when the contact surface is narrow, or a foreign material exists in the skin or the sensor. The error with respect to the temperature of the skin measured through the non-contact IR temperature sensor 604 may be smaller than the error with respect to the temperature of the skin measured through the contact temperature sensor.

In the embodiments described below, to perform the woman health service, it may be described that the processor 410 identifies the user's body temperature (or body temperature data) through the non-contact IR temperature sensor 604. However, it is not limited thereto. For example, the processor 410 may also identify a user's body temperature (or body temperature data) through a contact temperature sensor and perform a woman health service based on the identified body temperature.

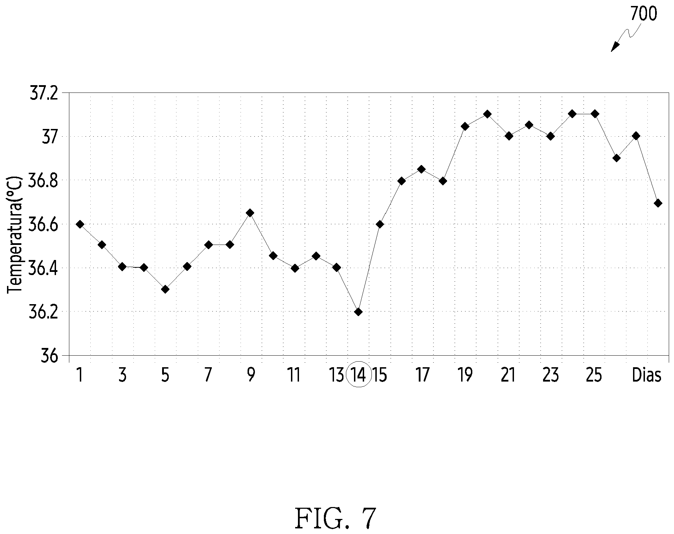

FIG. 7 is a diagram illustrating an example of an operation of a wearable device according to an embodiment.

Referring to FIG. 7, the processor 410 may identify an event (e.g., an ovulation date or a menstruation) related to a user (or a woman) based on the basal body temperature method. The processor 410 may identify a trend 700 in which the user's body temperature changes within a second cycle (e.g., one month or a menstrual cycle). For example, the processor 410 may identify the trend 700 in which the user's body temperature changes within the second cycle by identifying a lowest body temperature value during the day. As another example, the processor 410 may identify the trend 700 in which the user's body temperature changes within the second cycle by identifying the body temperature value at a designated time of day, or at a timing that satisfies a designated condition.

For example, the processor 410 may identify (or estimate) the ovulation date based on a change in body temperature before and after ovulation. For example, the processor 410 may identify (or estimate) the ovulation date through a decrease in body temperature by a first value (e.g., 0.3 degrees) immediately before ovulation, and an increase in body temperature by a second value (e.g., 0.5 degrees) after ovulation.

For example, based on the trend 700, the processor 410 may identify that the body temperature measured on the 14th day decreases by a first value and that the body temperature measured after 14 days increases by a second value. The processor 410 may identify the 14th day as the ovulation date.

An operation of the processor 410 for identifying an event related to a user may be described below based on the above-described basal body temperature method.

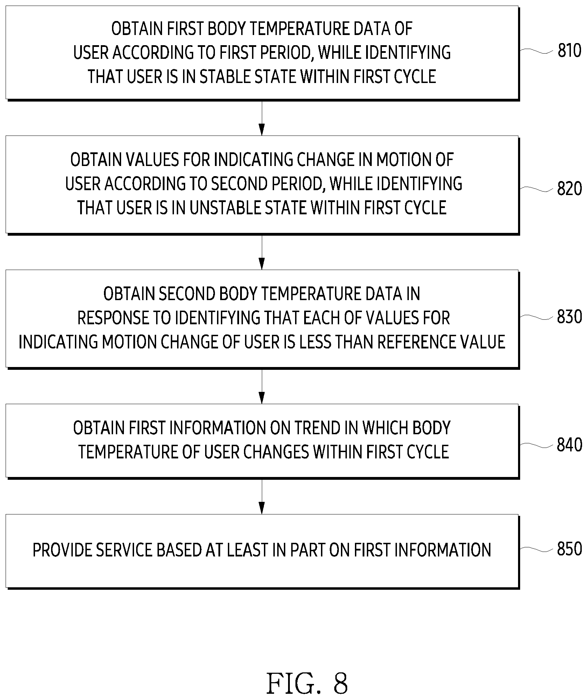

FIG. 8 is a flowchart illustrating an operation of a wearable device according to an embodiment. This method may be executed by the wearable device 400 and the processor 410 of the wearable device 400 illustrated in FIGS. 4 to 5.

Referring to FIG. 8, in operation 810, the processor 410 may obtain first body temperature data of the user according to the first period, while identifying that the user is in a stable state within the first cycle. For example the stable state is referred to a resting state.

For example, the stable state may include a sleep state. For another example, the stable state may include a meditation state, a motionless state (or a state in which a value for indicating a motion change is less than or equal to a designated value). For another example, the stable state may include a state in which the parasympathetic nerve of the user is activated.

For example, the processor 410 may identify that the user is in a stable state within the first cycle using at least one of a PPG sensor and/or a motion sensor.

For example, while identifying that the user is in a sleep state within the first cycle, the processor 410 may obtain the user's first body temperature data according to the first period. For example, while identifying using the PPG sensor 501 that the user is in a sleep state within the first cycle, the processor 410 may obtain the first body temperature data of the user according to the first period using the temperature sensor 502.

According to an embodiment, the processor 410 may identify that the user is in the sleep state within the first cycle. For example, the processor 410 may identify that the user is in the sleep state using the PPG sensor 501. For example, the processor 410 may obtain heart rate data through the PPG sensor 501. The processor 410 may identify that the user's heart rate is less than or equal to a designated range based on the heart rate data. The processor 410 may identify that the user is in a sleep state based on identifying that the user's heart rate is less than or equal to a designated range. According to an embodiment, the processor 410 may identify that the user is in the sleep state based on data identified through various sensors (e.g., motion sensor 503) as well as the PPG sensor 501. For example, the processor 410 may identify a value for the motion of the user through the motion sensor 503. The processor 410 may identify that the user is in the sleep state based on identifying that the identified value for the motion of the user is less than or equal to the reference value.

According to an embodiment, the processor 410 may obtain the first body temperature data of the user according to the first period. For example, the processor 410 may obtain the user's first body temperature data using the temperature sensor 502 according to the first period.

For example, the temperature sensor 502 may be disposed to be spaced apart from a part of the body of the user wearing the wearable device 400. The temperature sensor 502 may measure a temperature based on infrared light. For example, the temperature sensor 502 may include a non-contact IR temperature sensor 604.

For example, the processor 410 may obtain the first body temperature data of the user through the automatic measurement mode based on identifying that the user is in the sleep state. For example, the processor 410 may obtain the first body temperature data along a first period (e.g., 10 minutes to 30 minutes) shorter than the second period (e.g., 60 minutes), which is a measurement period in a non-sleep state. For example, the processor 410 may obtain the first body temperature data at a designated interval (e.g., 5 seconds) and a designated number of times (e.g., 3 times) according to the first period. According to an embodiment, the processor 410 may obtain the first body temperature data in response to identifying that the user is in a stable state through the motion sensor 503 according to the first period.

In operation 820, while identifying that the user is in an unstable state, the processor 410 may obtain values for indicating a change in motion of the user according to the second period. For example, the unstable state is referred to a non-resting state.

For example, the unstable state may include the non-sleep state. For example, the processor 410 may identify that the user is in an unstable state using at least one of the PPG sensor and/or the motion sensor.

For example, while identifying that the user is in the non-sleep state within the first cycle, the processor 410 may obtain values for indicating a change in motion of the user according to the second period. For example, while identifying using the PPG sensor 501 that the user is within the non-sleep state within the first cycle, the processor 410 may obtain values for indicating a change in motion of the user, using the motion sensor 503, according to a second period distinguished from the first period.

According to an embodiment, the processor 410 may identify whether the user is in an inactive state based on values for indicating the change in motion of the user obtained through the motion sensor 503 according to the second period. For example, the non-sleep state may include the inactive state and an active state. The inactive state may mean a state suitable for measuring body temperature through the temperature sensor 502. The active state may mean a state that is not suitable for measuring body temperature through the temperature sensor 502.

According to an embodiment, the processor 410 may set the second period to be longer than the first period. The second period may be set longer than the first period. For example, the first period may be set to 10 to 30 minutes. For example, the second period may be set to 30 minutes to 1 hour.

In operation 830, the processor 410 may obtain the second body temperature data in response to identifying that each of the values for indicating the motion change of the user is less than a reference value. The processor 410 may obtain the second body temperature data through the temperature sensor 502 in response to identifying that each of the values for indicating the motion change of the user is less than the reference value.

For example, the processor 410 may identify that values for indicating a motion change of the user obtained through the motion sensor 503 according to the second period are less than a reference value. The processor 410 may identify that the user is in the inactive state based on identifying that values for indicating a change in motion of the user are less than the reference value. In the non-sleep state, when the user is not in the inactive state, the obtained body temperature data may be inaccurate. Accordingly, the processor 410 may first identify that the user is in the inactive state according to the second period. When the user is in the inactive state, the processor 410 may obtain the second body temperature data using the temperature sensor 502.

According to an embodiment, the processor 410 may identify a user input while identifying that the user is in the non-sleep state within the first cycle. For example, the user input may be a user input for starting body temperature measurement. The processor 410 may obtain third body temperature data using the temperature sensor 502 based on the user input. Even when the user is in a drinking state, a fatigue state, a disease state, and/or an infected state, the processor 410 may obtain third body temperature data based on the user input. According to an embodiment, the processor 410 may determine an abnormality (e.g., high fever) of the user based on the third body temperature data.

In operation 840, the processor 410 may obtain first information on a trend in which the body temperature of the user changes within the first cycle. For example, the processor 410 may obtain the first information on the trend in which the body temperature of the user changes within the first cycle based on the first body temperature data and the second body temperature data.

For example, the processor 410 may set the first cycle to one day or 24 hours. The processor 410 may identify (or configure) a body temperature change graph over time based on the first body temperature data and the second body temperature data obtained for one day.

For example, the first information on a trend in which a user's body temperature changes within the first cycle may include first body temperature values obtained at a plurality of time points. The plurality of time points may be configured based on the first period or the second period within the first cycle. For example, while the user is in the sleep state, at least one time point may be configured based on the first period in the first cycle. While the user is in the non-sleep state, based on the second period within the first cycle, at least one other time point may be configured. The plurality of time points may include at least one time point and at least one other time point.

In operation 850, the processor 410 may provide a service based at least in part on the first information. For example, the processor 410 may perform a service-related function based at least in part on the first information.

The processor 410 may identify a trend (or information about the trend) in which the user's body temperature changes within a second cycle (e.g., one month or a menstrual cycle) based at least in part on the first information. The processor 410 may identify an event related to the user (e.g., ovulation date or menstruation day) based on a trend (or information on the trend) in which the user's body temperature changes within the second cycle. The processor 410 may provide a service based on the event related to the identified user. For example, the service may include services related to women's health. As an example, the service may include a menstruation date prediction service, an ovulation date confirmation service, a fertile window confirmation service, an abnormal symptom confirmation service, and/or a menarche prediction service.

In FIG. 8, an example of obtaining, in the sleep state, which is an example of a stable state, the user's first body temperature data, and an example of obtaining, in the non-sleep state, which is an example of an unstable state, values for indicating motion changes has been described, but it is not limited thereto. The processor 410 may obtain the first body temperature data of the user even when the user is meditating or does not move.

In the following description, an operation for providing a service may be described in order based on an operation for obtaining (or identifying) information on the trends in which the user's body temperature changes within the first cycle(e.g., 1 day), an operation for obtaining (or identifying) information on the trend in which the user's body temperature changes within the second cycle (e.g. one month or menstrual cycle), and information on the trend in which the user's body temperature changes within the second cycle.

First, hereinafter, an operation for identifying information on a trend in which a user's body temperature changes within the first cycle may be described.

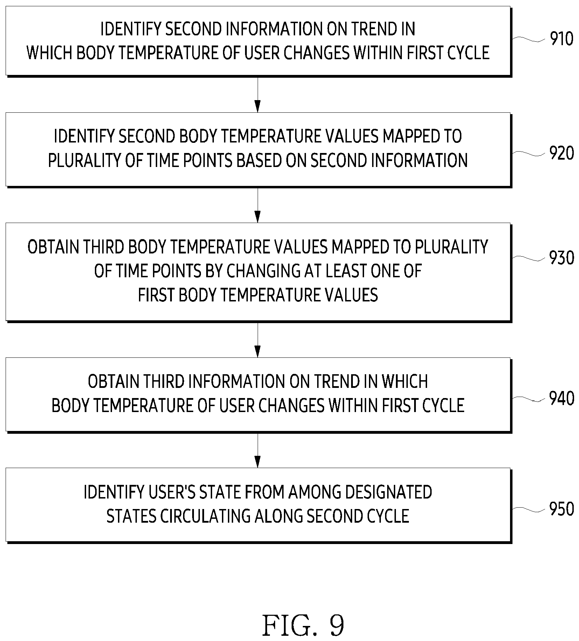

According to an embodiment, the processor 410 may obtain body temperature data (or a plurality of body temperature values) of the user at a plurality of time points in order to obtain first information on a trend in which the body temperature of the user changes in the first cycle. The body temperature data of the user obtained at the plurality of time points may include an error value (or an outlier). By correcting the error value, the processor 410 may obtain the first information on a trend in which the body temperature of the user changes in the first cycle. An embodiment of obtaining, by correcting the error value, the first information on a trend in which the body temperature of the user changes in the first cycle may be described in FIGS. 9 to 15.

FIG. 9 is another flowchart illustrating an operation of a wearable device according to an embodiment. This method may be executed by the wearable device 400 and the processor 410 of the wearable device 400 illustrated in FIGS. 4 to 5.

Referring to FIG. 9, in operation 910, the processor 410 may identify second information on a trend in which a user's body temperature changes in the first cycle. For example, the processor 410 may identify the second information on a trend in which a user's body temperature changes in the first cycle stored in the memory 440 of the wearable device 400.

For example, the processor 410 may obtain first information on a trend in which a user's body temperature changes within the first cycle. For example, the processor 410 may obtain the first information on a trend in which a user's body temperature changes within the first cycle, by performing operation 840 of FIG. 8.

The processor 410 may identify second information on a trend in which a user's body temperature changes within the first cycle, stored in the memory 440 after obtaining the first information. Before the first information is obtained, the processor 410 may obtain the second information based on the obtained body temperature data. The processor 410 may store the obtained second information in the memory 440. After obtaining the first information, the processor 410 may identify the second information stored in the memory 440. For example, the second information may be related to a trend in which the body temperature of the user changes within the first cycle, patterned according to the repeated first cycle.

In operation 920 , the processor 410 may identify second body temperature values mapped to a plurality of time points based on the second information. The trend in which the user's body temperature changes within the first cycle according to the second information may be configured to second body temperature values according to a plurality of time points. For example, the trend may include a graph of a change in body temperature of a user during a day. Accordingly, the processor 410 may identify the graph of a change in body temperature of a user during a day stored in the memory 440. The graph may be configured to second body temperature values mapped to the plurality of time points.

In operation 930, the processor 410 may change at least one of the first body temperature values to obtain third body temperature values mapped to a plurality of time points. For example, the processor 410 may identify a critical range for the user's body temperature based on the second information. The processor 410 may identify at least one of the first body temperature values outside the identified critical range. The processor 410 may obtain third body temperature values mapped to a plurality of time points by changing at least one of the identified first body temperature values. For example, the processor 410 may identify at least one of the obtained first body temperature values as at least one error value (or outlier). The processor 410 may obtain third body temperature values mapped to a plurality of time points by changing at least one error value. For example, the standard deviation value of the third body temperature values with respect to the second body temperature values may be less than the standard deviation value of the first body temperature values with respect to the second body temperature values.

In operation 940, the processor 410 may obtain third information on a trend in which the body temperature of the user changes within the first cycle. For example, the processor 410 may obtain the third information on a trend in which a user's body temperature changes within the first cycle based on the third body temperature values. For example, the third information may be obtained by changing the first information based on the second information stored in the memory 440. The processor 410 may obtain the third information by changing the first information based on the second information stored in the memory 440.

According to an embodiment, the processor 410 may update a trend in which a user's body temperature changes within the first cycle according to the second information based on the second information and the third information.

In operation 950, the processor 410 may identify a user's state from among designated states circulating along the second cycle. For example, the processor 410 may identify the user's state among designated states circulating along the second cycle exceeding the first cycle based on the second information and the third information.

According to an embodiment, the processor 410 may identify fourth body temperature values satisfying a designated condition along the first cycle based on the second information and the third information. For example, the processor 410 may identify a fourth body temperature value having a minimum value from among a plurality of body temperature values within the first cycle based on the second information and the third information. The processor 410 may identify fourth body temperature values along the first cycle. For example, the processor 410 may identify fourth body temperature values along the first cycle within the second cycle. For example, the processor 410 may identify a fourth body temperature value that is a minimum value of body temperature during a day and identify fourth body temperature values for a month.

According to an embodiment, the processor 410 may obtain the fourth information on a trend in which a user's body temperature changes within the second cycle based on the fourth body temperature values. For example, the processor 410 may obtain the fourth information on a trend in which a user's body temperature changes within the second cycle exceeding the first cycle based on the fourth body temperature values. For example, the first cycle may be set to 1 day. The second cycle may be set to one month (or menstrual cycle). The processor 410 may obtain fourth information on a trend in which a user's body temperature changes for a month.

According to an embodiment, the processor 410 may identify a user's state among designated states circulating along the second cycle. For example, the processor 410 may identify a user's state among designated states circulating along the second cycle based on the fourth information.

For example, the processor 410 may identify a change in the user's body temperature within the second cycle based on the fourth information on the trend in which the user's body temperature changes within the second cycle.

For example, the processor 410 may identify the user's state as one of the designated states according to the basal body temperature method based on the fourth information. The designated states may be cycled along the second cycle. All designated states may be included in the second cycle. Based on the second cycle being repeated, the designated states may cycle. As an example, the designated states may include menstrual phase status, follicular phase status, ovulation phase status, and luteal phase status.

According to an embodiment, the processor 410 may provide a service (e.g., a woman health service) by identifying a user's state among designated states circulating along the second cycle.

Hereinafter, an example of an operation of the wearable device according to operations 910 to 950 may be described. Hereinafter, for convenience of description, a trend in which a user's body temperature changes within a first cycle according to the first information may be described as a circadian rhythm. A trend in which the user's body temperature changes within the first cycle according to the second information may be described as a baseline (or reference bio-rhythm). A trend in which a user's body temperature changes within the first cycle according to the third information may be described as a corrected circadian rhythm. In addition, the first period may be set to 1 day (i.e., 24 hours).

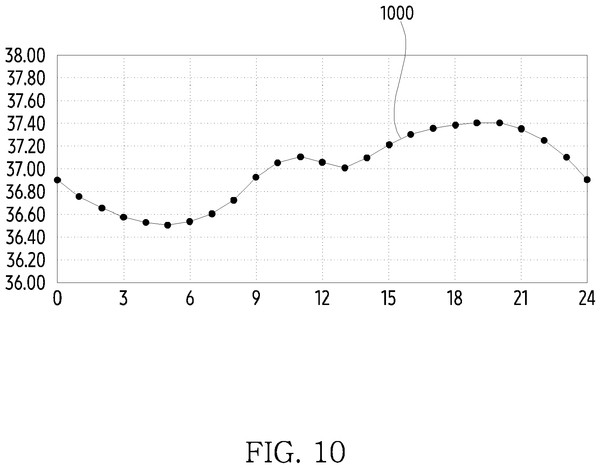

FIG. 10 is a diagram illustrating an example of an operation of a wearable device according to an embodiment.

Referring to FIG. 10, the processor 410 may obtain a circadian rhythm 1000 of a user. The user's circadian rhythm 1000 may represent a change in the user's body temperature for 24 hours. In general, the lowest body temperature may be identified during the morning (e.g., 6 o'clock). The highest body temperature may be identified during the afternoon (e.g., 17:00). Accordingly, the processor 410 may identify that the time at which the minimum body temperature of the user is identified is 5 o'clock based on the circadian rhythm 1000. The processor 410 may identify that the time at which the maximum body temperature of the user is identified is 19 o'clock based on the circadian rhythm 1000.

According to an embodiment, the processor 410 may identify whether the user has eaten based on the circadian rhythm 1000. The processor 410 may identify whether the user's state is a stable state or an unstable state based on the circadian rhythm 1000. For example, the processor 410 may identify whether the user's state is a sleep state or a non-sleep state based on the circadian rhythm 1000.

In general, the body temperature of the user may increase due to metabolic activities through meals. The processor 410 may identify whether the user has eaten based on identifying that the user's body temperature increases. According to an embodiment, in the case of a user requiring diet or blood sugar management, the processor 410 may perform a blood sugar monitoring operation using a blood sugar sensor in an inactive state after the user eats. For example, the processor 410 may monitor the user's blood sugar level at a designated period (e.g., 10 minutes). The processor 410 may obtain the user's body temperature value for each time of designated time together, from the time point when the user's blood sugar level is suddenly changed to high until it is changed to the average blood sugar level. The processor 410 may identify (or manage, monitor) whether the user has eaten with an appropriate diet and/or whether there is no problem in the metabolism process of digestion by obtaining the user's body temperature value and blood sugar level at a period of designated time.

In general, metabolic activity may decrease in a sleep state. Accordingly, according to an embodiment, the processor 410 may identify that the user is in a sleeping state based on identifying that the user's body temperature is reduced. The processor 410 may identify the first body temperature data of the user according to the first period while the user is in the sleep state. While the user is in the non-sleep state, the processor 410 may identify that the user is in an inactive state according to the second period, and may identify the second body temperature data of the user. For example, the second period may be set to be longer than the first period.

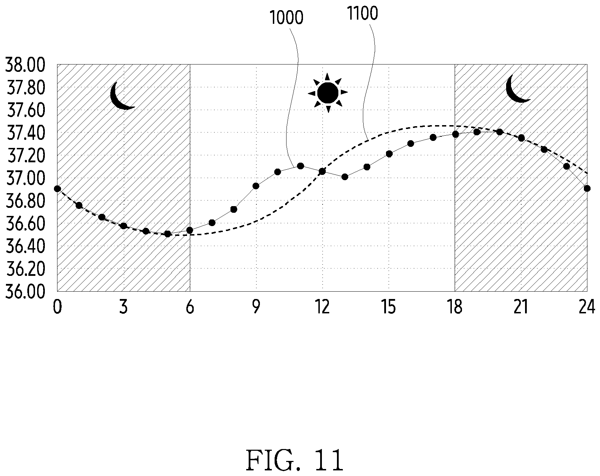

FIG. 11 is a diagram illustrating another example of an operation of a wearable device according to an embodiment.

Referring to FIG. 11, the processor 410 may identify the baseline 1100 based on the circadian rhythm 1000 of the user.

According to an embodiment, the processor 410 may identify (or measure and obtain) a user's body temperature value (or body temperature data) in a non-sleep state for 24 hours through the temperature sensor 502. The processor 410 may identify (or measure, obtain) the user's body temperature value (or body temperature data) according to the user's main activity (e.g., eating or exercising). The processor 410 may identify (or measure, obtain) the user's body temperature value (or body temperature data) based on a user input (or a user request) received from the user. According to an embodiment, the processor 410 may identify (or measure or obtain) the user's body temperature value (or body temperature data) in a sleep state.

The processor 410 may identify (or obtain) the circadian rhythm 1000 of the user based on the identified body temperature values. The processor 410 may obtain the baseline 1100 by processing (or analyzing) the circadian rhythm 1000 of the user. For example, the baseline 1100 may be identified based on a minimum body temperature value and a maximum body temperature value of the circadian rhythm 1000 of the user. For example, the body temperature value of the user identified in the non-sleep state may be used to identify the baseline 1100. For example, the user's body temperature value according to the user's main activity may be used to identify the critical range of the baseline 1100.

According to an embodiment, the processor 410 may store the baseline 1100 identified together with the circadian rhythm 1000 in the memory 440. According to an embodiment, the processor 410 may store heart rate (HR) data, heart rate variability (HRV) data, activity time data, or sleep time data together with the baseline 1100 in the memory 440. For example, the main activity history of the user over time may be stored together in the baseline 1100.

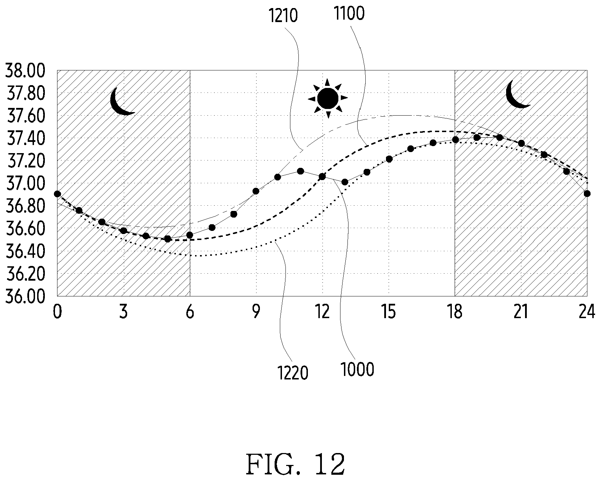

FIG. 12 is a diagram illustrating another example of an operation of a wearable device according to an embodiment.

Referring to FIG. 12, the processor 410 may correct (or change) the baseline 1100 according to the user's sleep state.

According to an embodiment, the processor 410 may identify the circadian rhythm 1000 during a day (or for 24 hours) of the user. The processor 410 may identify the baseline 1100 based on the circadian rhythm 1000. The processor 410 may identify the circadian rhythms of the user every day. The processor 410 may correct (or change) the baseline 1100 based on the circadian rhythms of the user identified every day. The processor 410 may identify the baseline 1100 according to the user based on the circadian rhythms of the user identified every day.

For example, the processor 410 may correct an offset based on information on the user's sleep pattern and the user's circadian logical rhythms (or body temperature value, body temperature data) identified every day. For example, the information on the user's sleep pattern may include information on the bedtime, information on the quality of sleep, and/or information on the total sleep time.

For example, when the user's bedtime is delayed or the user cannot sleep deeply, the circadian rhythm may change. When the baseline 1100 is maintained the same even though the circadian rhythm is changed, most body temperature values may be identified as error values. Accordingly, the processor 410 may correct the offset based on information on the user's sleep pattern and the user's circadian rhythms (or body temperature value, body temperature data) identified every day.

For example, the processor 410 may change the baseline 1100 to the baseline 1210 based on identifying that the user does not get deep sleep. For another example, the processor 410 may change the baseline 1100 to the baseline 1220 based on identifying that the user has deep sleep.

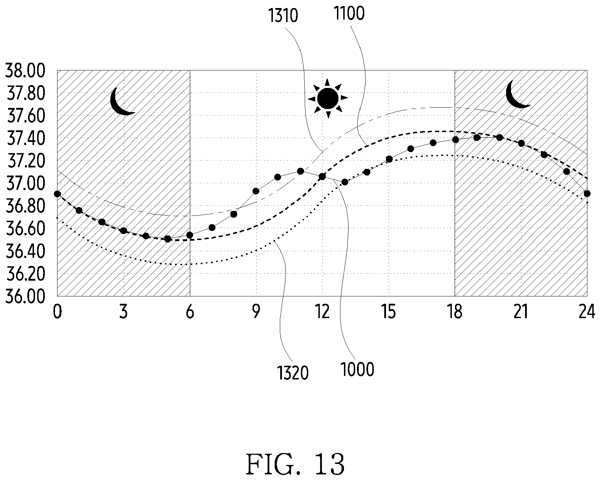

FIG. 13 is a diagram illustrating another example of an operation of a wearable device according to an embodiment.

Referring to FIG. 13, the processor 410 may obtain a body temperature value (e.g., third body temperature data of FIG. 8) based on a user input (or a user's request). The processor 410 may use the body temperature value identified based on the user input to identify the reference line. The processor 410 may identify the state of the user based on the identification of the user's body temperature value exceeding the reference line or less than the reference line.

For example, the processor 410 may identify a body temperature value while the user is in a main activity. For another example, the processor 410 may identify a body temperature value based on a user input in a state in which the user's state is designated. The designated state may include a fever state, a mild fever state, a disease state, and/or an infected state.

For example, the processor 410 may identify the first line 1310 based on the identified body temperature value when the user's state is a first state (e.g., a fever state). After the first line 1310 is identified, the processor 410 may identify a user's body temperature value. The processor 410 may identify that the user's state is the first state based on the user's body temperature value exceeding the first line 1310.

For another example, the processor 410 may identify the second line 1320 based on the identified body temperature value when the user's state is a second state (e.g., a low body temperature state). After the second line 1320 is identified, the processor 410 may identify a user's body temperature value. The processor 410 may identify that the user's state is the second state based on the user's body temperature value being less than the second line 1320.

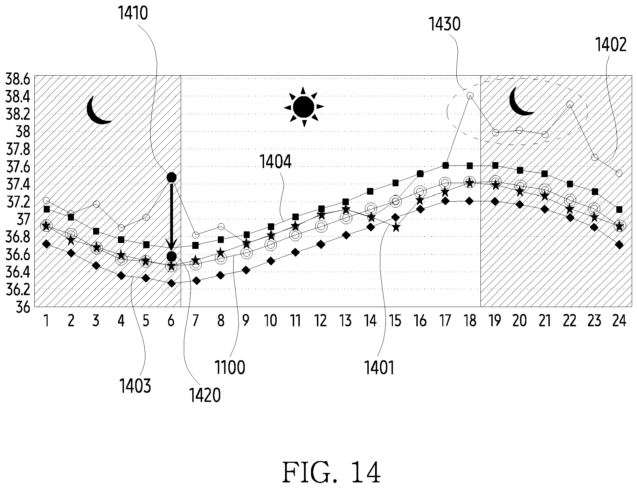

FIG. 14 is a diagram illustrating another example of an operation of a wearable device according to an embodiment.

Referring to FIG. 14, the processor 410 may obtain third information on a trend in which a user's body temperature changes within a first cycle by performing operations 930 and 940 of FIG. 9. For example, the processor 410 may change at least one of the first body temperature values included in the first information and obtained at a plurality of points in the first cycle. The processor 410 may obtain third information on the corrected trend by changing at least one of the first body temperature values.

According to an embodiment, the processor 410 may identify a critical range for the user's body temperature based on the second information. For example, the processor 410 may identify the critical range as a section between the first critical line 1403 and the second critical line 1404.

According to an embodiment, the processor 410 may identify the first critical line 1403 and the second critical line 1404 based on the baseline 1100. The processor 410 may identify the first critical line 1403 and the second critical line 1404 as a section within a range designated in the baseline 1100. For example, the processor 410 may identify the first critical line 1403 by moving the baseline 1100 downward by a designated value (e.g., 0.4 degrees). The processor 410 may identify the second critical line 1404 by moving the baseline 1100 upward by a designated value (e.g., 0.4 degrees).

According to an embodiment, the processor 410 may identify the circadian rhythm 1401 on the first day. The processor 410 may identify that the circadian rhythm 1401 is within a critical range. The processor 410 may identify that the body temperature values of the circadian rhythm 1401 are less than or equal to the first critical line 1403. The processor 410 may identify that the chain values of the circadian rhythm 1401 are equal to or greater than the second critical line 1404.

According to an embodiment, the processor 410 may identify the circadian rhythm 1402 on the second day. The processor 410 may identify that the body temperature value 1410 exceeds the second critical line 1404. The processor 410 may identify that the body temperature value 1410 is an error value. For example, the processor 410 may identify that the body temperature value 1410 is an error value based on the circadian rhythm algorithm. For another example, the processor 410 may identify the body temperature value 1410 as an error value by comparing the body temperature value 1410 with the body temperature values around the identified time. The processor 410 may change (or correct) the body temperature value 1410 identified as the error value to the body temperature value 1420.

According to an embodiment, the processor 410 may identify that the body temperature values 1430 exceed the first critical line 1403 and exceed the reference line (not shown) of the fever state. The processor 410 may identify that the user is in the fever state in a time interval in which the body temperature values 1430 are identified.

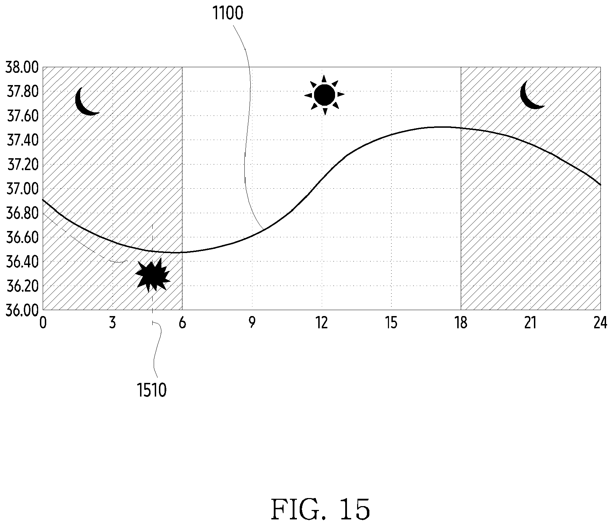

FIG. 15 is a diagram illustrating another example of an operation of a wearable device according to an embodiment.

Referring to FIG. 15 , the processor 410 may not be able to identify the user's body temperature value after a time point 1510. For example, the processor 410 may not identify the user's body temperature due to deterioration of hardware performance of the wearable device 400 or battery discharge.

After the time point 1510, even when the user's body temperature value is not identified, the processor 410 may identify (or estimate) a trend in which the user's body temperature changes based on the previously identified body temperature value in a state of similar users or the baseline 1100.

According to an embodiment, the processor 410 may repeatedly obtain circadian rhythms every day. The processor 410 may correct (or enhance) the baseline 1100 based on the obtained circadian logical rhythms. For example, the processor 410 may identify a user-specific (or individual) baseline by calibrating (or enhancing) the baseline 1100 during a second cycle (e.g., one month or a menstrual cycle). For example, the processor 410 may identify the baseline 1100 according to the user based on the circadian rhythm patterning.

According to an embodiment, the processor 410 may identify an accurate body temperature value of the user by repeating an operation of correcting (or enhancing) the baseline 1100. Accordingly, by identifying an accurate body temperature value of the user, the processor 410 may accurately identify the menstrual cycle, the fertile window (or ovulation date), and/or the contraceptive period.

The above-described embodiments are embodiments for obtaining (or identifying) information on a trend in which a user's body temperature changes within a first cycle (e.g., 1 day). Hereinafter, an embodiment for obtaining (or identifying) information on a trend in which a user's body temperature changes within a second cycle (e.g., one month) may be described.

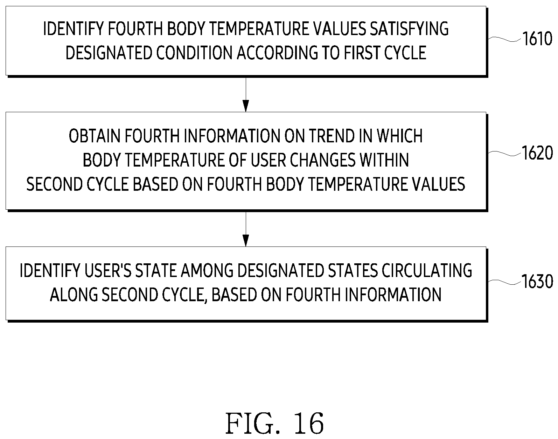

FIG. 16 is another flowchart illustrating an operation of a wearable device according to an embodiment. This method may be executed by the wearable device 400 and the processor 410 of the wearable device 400 illustrated in FIGS. 4 to 5. Operations 1610 to 1630 of FIG. 16 may be related to operation 950 of FIG. 9.

Referring to FIG. 16, in operation 1610, the processor 410 may identify fourth body temperature values satisfying a designated condition according to a first cycle. For example, the processor 410 may identify fourth body temperature values satisfying a designated condition according to the first cycle based on the second information and the third information. For example, the second information and the third information may correspond to the second information and the third information described in FIG. 9.

According to an embodiment, the fourth body temperature value satisfying the designated condition within the first cycle may refer to a body temperature value having a minimum body temperature value within the first cycle. Accordingly, the processor 410 may identify a body temperature value having a minimum body temperature value of the user within the first cycle as the fourth body temperature value. The processor 410 may identify the fourth body temperature values according to the first cycle repeated within the second cycle. For example, the processor 410 may identify a minimum body temperature value from among body temperature values identified for one day as the fourth body temperature value. The processor 410 may identify the fourth body temperature values by identifying the fourth body temperature values for a month. For example, the fourth body temperature value, which is the minimum of the body temperature values identified during the day, may be referred to as a basal body temperature value (or basal body temperature).

In operation 1620, the processor 410 may obtain the fourth information on a trend in which the user's body temperature changes within the second cycle based on the fourth body temperature values. For example, the processor 410 may obtain a graph of daily body temperature changes within the second cycle. For example, the processor 410 may obtain a graph of daily body temperature changes during a month (or menstrual cycle).

In operation 1630, the processor 410 may identify the user's state among designated states circulating along the second cycle, based on the fourth information. The processor 410 may provide a service (e.g., a woman health service) based on the identified state of the user. For example, the processor 410 may identify the user's state as one of menstrual phase status, follicular phase status, ovulation phase status, and luteal phase status based on a daily body temperature change. For example, the processor 410 may divide the second cycle (e.g., a month or a menstrual cycle) into time intervals corresponding to designated states.

According to an embodiment, the processor 410 may pattern a trend in which a user's body temperature changes within the second cycle as the second cycle is repeated. For example, as the second cycle is repeated, the processor 410 may identify an accurate body temperature value of the user changed in the second cycle by repeating an operation of correcting (or enhancing) a trend in which the body temperature of the user changes within the second cycle. The processor 410 may accurately identify the menstrual cycle, fertile window (or ovulation date), and/or contraceptive period by identifying the exact temperature value of the user that changes within the second cycle.

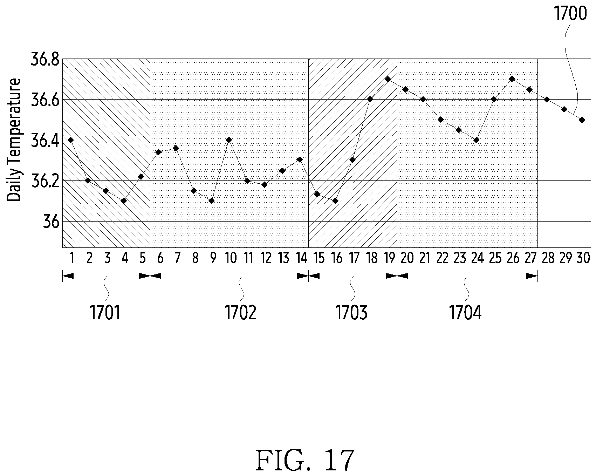

FIG. 17 is a diagram illustrating another example of an operation of a wearable device according to an embodiment.

Referring to FIG. 17, the processor 410 may identify (or obtain) a graph 1700 indicating a change in daily body temperature during a menstrual cycle (or one month). For example, the processor 410 may identify (or obtain) a graph 1700 indicating a daily body temperature change for a month based on the body temperature values that are the minimum of the body temperature values for a day.

According to an embodiment, the processor 410 may divide the menstrual cycle into a period 1701, a period 1702, a period 1703, and a period 1704 based on the basal body temperature method. For example, the period 1701 may mean a menstruation phase state. The period 1702 may mean a follicular (or follicular phase) state. The period 1703 may mean an ovulation phase state. The period 1704 may refer to a luteal phase state. For example, the processor 410 may identify a period including a minimum body temperature value and a maximum body temperature value as an ovulation phase state based on the identification that the difference between the minimum body temperature value and the maximum body temperature value within the menstrual cycle is greater than or equal to a designated value (e.g., 0.5 to 1 degree). For another example, the processor 410 may identify a period in which a temperature change occurs more than or equal to a designated value within the menstrual cycle as the ovulation phase.

According to an embodiment, the processor 410 may identify average values of body temperature values in each of periods 1701 to 1704. The processor 410 may identify the accuracy of the ovulation date based on the average values of the body temperature values of the periods 1701 to 1704.

For example, the processor 410 may identify a first average value and a first standard deviation of body temperature values in the period 1701. The processor 410 may identify a second average value and a second standard deviation of body temperature values in the period 1702. The processor 410 may identify a third average value and a third standard deviation of body temperature values in the period 1703. The processor 410 may identify a fourth average value and a fourth standard deviation of body temperature values in the period 1704. The difference between the second average value identified in the follicular (or follicular phase) state period 1702 and the fourth average value identified in the luteal phase state period 1704 may be identified to be a designated value (e.g., 0.5 to 1 degree). Accordingly, the processor 410 may identify that as the difference between the second average value and the fourth average value is greater than the second standard deviation to the fourth standard deviation, the accuracy of the identified ovulation phase period 1703 is higher.

A specific example of the above-described embodiment may be described in Table 1.

Table 1 shows the average value and the standard deviation value in each section.

**[Table 1]**

| Daily Temperature | follicular phase | ovulation phase | luteal phase |
|---|---|---|---|
| average | 36.23°C/97.21F | 36.37°C/97.47F | 36.57°C/97.83F |
| STDEV | 0.10°C/0.18F | 0.27°C/0.49F | 0.11°C/0.20F |
| Temperature shift | | 0.34°C/0.61F | |
| Temperature shift / STDEV | 3.35°C/6.03F | 1.25°C/2.25F | 3.18°C/5.72F |

Referring to Table 1, the processor 410 may identify an average value of body temperature values in the follicular phase as 36.23 degrees. The processor 410 may identify the standard deviation of the body temperature values in the follicular phase as 0.10. The processor 410 may identify the average value of the body temperature values in the ovulation phase as 36.37 degrees. The processor 410 may identify the standard deviation of the body temperature values in the ovulation phase as 0.27. The processor 410 may identify an average value of the body temperature values in the luteal phase at 36.57 degrees. The processor 410 may identify the standard deviation of the body temperature values in the luteal phase as 0.11. The processor 410 may identify a difference (hereinafter, a temperature difference) between the average values of the body temperature values in the ovulation phase and the average values of the body temperature values in the follicular phase as 0.34 degrees. The processor 410 may identify a first value obtained by dividing a temperature shift by the standard deviation of the follicular phase as 3.35. The processor 410 may identify the second value obtained by dividing the temperature shift by the standard deviation of the ovulation phase as 1.25. The processor 410 may identify a third value obtained by dividing the temperature shift by the standard deviation of the luteal phase as 3.18. The processor 410 may identify the accuracy of the identified ovulation phase (or ovulation date) based on the first to third values. For example, the processor 410 may identify the identified ovulation phase (or ovulation date) with higher accuracy as the first to third values are higher.

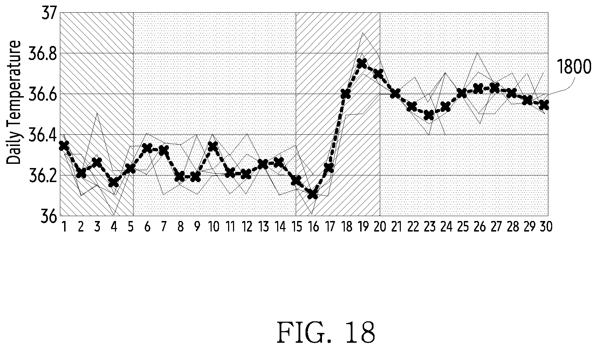

FIG. 18 is a diagram illustrating another example of an operation of a wearable device according to an embodiment.

Referring to FIG. 18 , FIG. 18 is a diagram illustrating accumulated trends in which a user's body temperature changes according to a second cycle. The processor 410 may identify trends in which a user's body temperature is changed according to a second cycle (e.g., a month or a menstrual cycle). The processor may identify the patterned trend 1800 within the second cycle by patterning the identified trends. Whenever the second cycle is repeated, the processor 410 may correct (or enhance) the patterned trend 1800. The processor 410 may provide a service to a user based on the patterned trend 1800. The processor 410 may correct (or change) the identified basal body temperature value based on the patterned trend 1800. The processor 410 may accurately identify the ovulation phase (or ovulation date) by correcting (or changing) the identified basal body temperature value.

For example, when a trend in which a user's body temperature changes within the second cycle is identified, the processor 410 may identify whether the user has an abnormal symptom by analyzing the trend based on the patterned trend 1800. For another example, the processor 410 may analyze the trend based on the patterned trend 1800 to identify a time point at which an event related to a user will be occurred.

The above-described embodiments are embodiments for obtaining (or identifying) information on a trend in which a user's body temperature changes within a second cycle (e.g., one month). Hereinafter, based on information about trends in which the user's body temperature changes within the second cycle, an embodiment for providing a service by the processor 410 may be described.

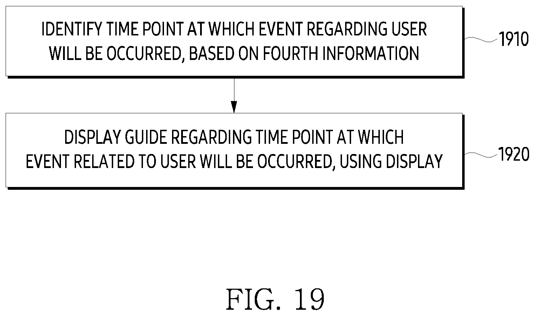

FIG. 19 is a flowchart illustrating an operation of a wearable device according to an embodiment. This method may be executed by the wearable device 400 and the processor 410 of the wearable device 400 illustrated in FIGS. 4 to 5.

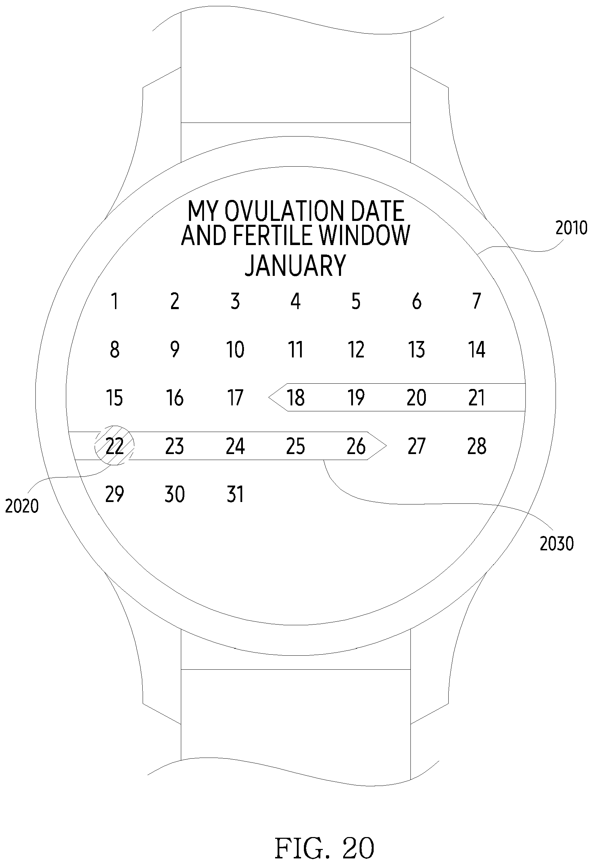

FIG. 20 is a diagram illustrating another example of an operation of a wearable device according to an embodiment.

Referring to FIG. 19, in operation 1910, the processor 410 may identify a time point at which an event regarding a user will be occurred based on the fourth information. For example, based on the fourth information on the trend in which the body temperature of the user changes within the second cycle, the processor 410 may identify a time point at which an event regarding the user will be occurred. For example, the processor 410 may identify an ovulation date based on a trend in which a body temperature of a user (or a woman) changes for one month.

According to an embodiment, the processor 410 may identify a user's state among designated states circulating for one month based on a trend in which the body temperature of the user (or woman) changes for one month. For example, the processor 410 may identify the user's state as one of menstrual phase state, follicular phase status, ovulation phase status, and luteal phase state based on a trend in which the user's body temperature changes for a month.

For example, the processor 410 may identify an ovulation date based on the basal body temperature method. Even when the user has an irregular menstrual cycle, the processor 410 may identify the ovulation date.

In operation 1920, the processor 410 may display a guide regarding a time point at which an event related to the user will be occurred using the display 420. For example, the processor 410 may display a guide by emphasizing a visual object corresponding to a time point at which an event regarding the user will be occurred.

Referring to FIG. 20, the processor 410 may display a guide regarding a time point at which an event related to a user will be occurred on the screen 2010. The processor 410 may display a guide for the user's ovulation date and fertile window on the screen 2010. Although not shown, the processor 410 may display a guide for when various women's health-related events such as a menstrual start date and a menstrual end date will be occurred, as well as an ovulation date and fertile window.

The processor 410 may display a visual object 2020 indicating an ovulation date on the screen 2010. The visual object 2020 indicating the ovulation date may be highlighted and displayed more than visual objects corresponding to other dates. The processor 410 may display a visual object 2030 indicating fertile window on the screen 2010.

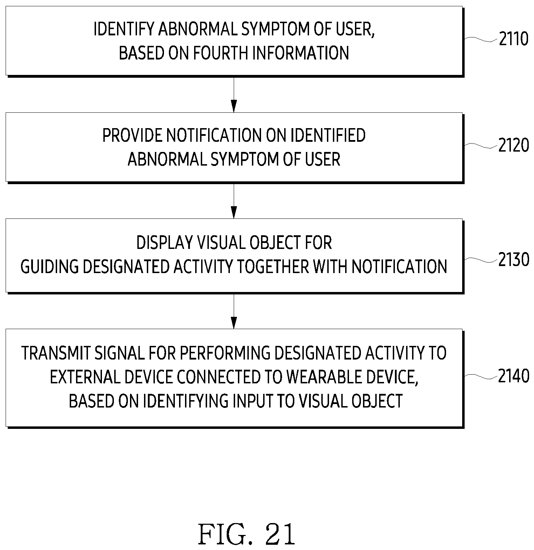

FIG. 21 is a flowchart illustrating an operation of a wearable device according to an embodiment. This method may be executed by the wearable device 400 and the processor 410 of the wearable device 400 illustrated in FIGS. 4 to 5.

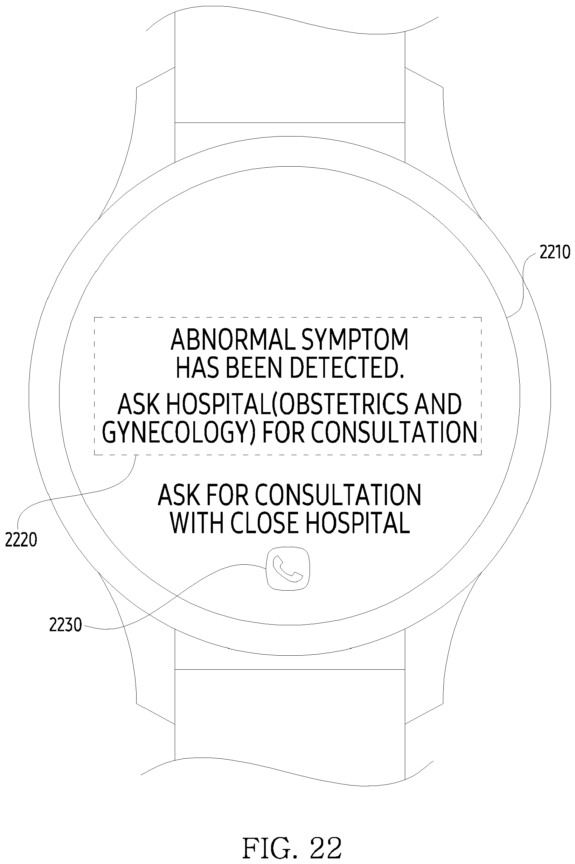

FIG. 22 is a diagram illustrating another example of an operation of a wearable device according to an embodiment.

Referring to FIG. 21, in operation 2110, the processor 410 may identify an abnormal symptoms of a user based on the fourth information.

According to an embodiment, the processor 410 may identify whether a user's duration of flow and/or a menstrual cycle is normal or abnormal. For example, the processor 410 may identify that the user's menstrual duration of flow is abnormal based on the user's duration of flow being out of a first designated period (e.g., 4 to 6 days). As another example, the processor 410 may identify that the user's menstrual cycle is abnormal based on the user's menstrual cycle outside the second designated period (e.g., 21 to 35 days).

For example, processor 410 may identify that the user is likely to have abnormal bleeding, uterine myoma, adenomyoma, endometrial structural abnormalities, thyroid dysfunction, and/or hormonal imbalance based on identifying an abnormality in the user's menstrual period and/or menstrual cycle.

According to an embodiment, the processor 410 may identify that the menstrual cycle of a user having a normal menstrual cycle is rapidly changed. The processor 410 may identify abnormal symptom of the user based on identifying that the user's menstrual cycle is rapidly changed. For example, the processor 410 may identify that the user does not menstruate for a designated number of times (e.g., 3 time) cycles (e.g., 3 months). As another example, the processor 410 may identify that the user's menstrual cycle is irregular for a designated time (e.g., 3 months).

For example, the processor 410 may identify that the user is likely to have amenorrhea and/or a menstrual disorder based on identifying a sudden change in the menstrual cycle of a user who had a normal menstrual cycle.

For another example, the processor 410 may identify that the trend in which the user's body temperature changes is different from the trends previously identified through the cycle. In addition, the processor 410 may identify that the user is likely to have menopause and/or menopause based on identifying that the user's menstrual cycle is rapidly changed. In addition, the processor 410 may identify that the user is likely to have climacterium and/or menopause based on identifying that the user's menstrual cycle is rapidly changed.

In operation 2120, the processor 410 may provide a notification on the identified abnormal symptom of the user. For example, processor 410 may provide a notification of the identified user's abnormal symptom based on the identified user's abnormal symptom.

For example, the processor 410 may provide a notification by displaying a text indicating that an abnormal symptom of the user has occurred on the display 420. For another example, the processor 410 may provide a notification by outputting a sound indicating that an abnormal symptom of the user has occurred.

In operation 2130, the processor 410 may display a visual object for performing a designated activity together with a notification. For example, the processor 410 may display a visual object for performing a designated activity according to an abnormal symptom of a user. For example, the processor 410 may display a visual object for performing a telephone connection to a hospital. As another example, the processor 410 may display a visual object for performing an online medical service.

In operation 2140, the processor 410 may transmit a signal for performing a designated activity to an external device connected to the wearable device based on identifying an input to the visual object.

For example, the processor 410 may transmit a signal for performing a telephone connection to the hospital to an external device. The external device may be used to perform a telephone connection. The external device may perform a telephone connection to the hospital based on the signal.

For another example, the processor 410 may transmit a signal for performing an online medical service to an external device. The external device may be used to perform an online medical service. The external device may perform an online medical service based on the signal.

Referring to FIG. 22, the processor 410 may provide a notification of abnormal symptoms of a user to the screen 2210. The processor 410 may provide a notification by displaying a text 2220 indicating an abnormal symptom of a user.

The processor 410 may display a visual object 2230 for performing a designated activity together with a text 2220 indicating an abnormal symptom of a user. For example, the processor 410 may display a visual object 2230 for performing a telephone connection to a close hospital. The processor 410 may transmit a signal for performing a telephone connection to a close hospital to an external device connected to the wearable device 400 based on identifying an input to the visual object 2230. For example, the input to the visual object 2230 may be variously set. The input can be set to at least one of a tab input, a double tab input, and a swipe input.

For example, a signal for performing a phone connection to a close hospital may include a control signal for executing a phone application of an external device. The processor 410 may control the external device to execute a phone application and to perform a phone connection to a nearby hospital by transmitting the above signal to an external device. As another example, the processor 410 may display a visual object for transmitting information on the identified abnormal symptoms of the user to an external device. The external device may be used to perform an online medical service. The external device may perform an online medical service based on the signal.

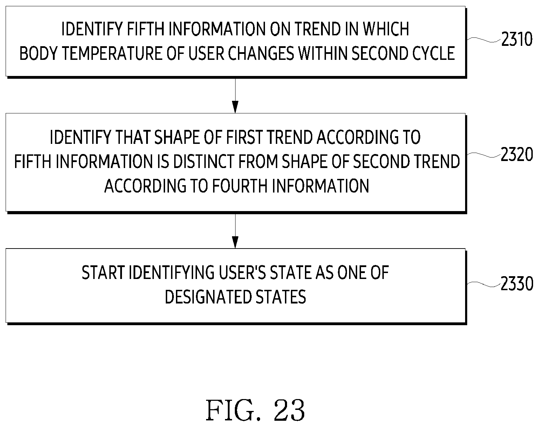

FIG. 23 is a flowchart illustrating an operation of a wearable device according to an embodiment. This method may be executed by the wearable device 400 and the processor 410 of the wearable device 400 illustrated in FIGS. 4 to 5.

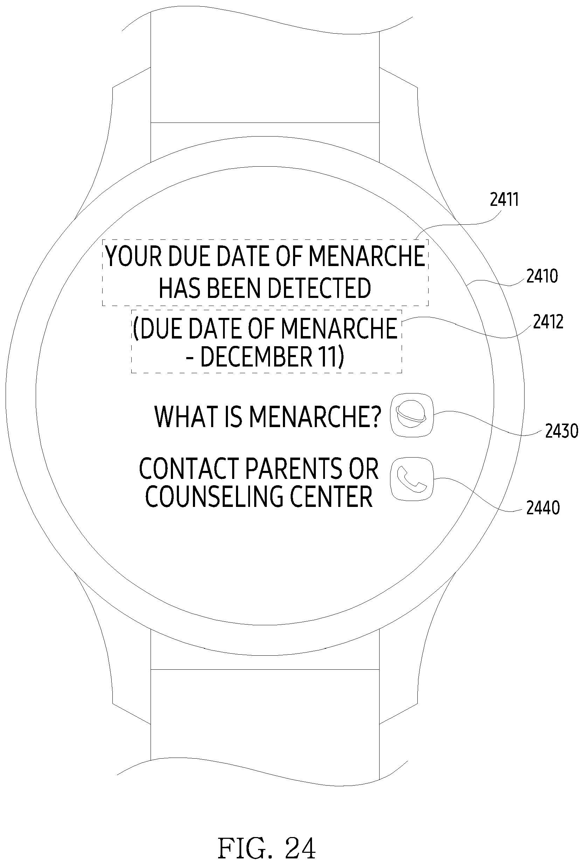

FIG. 24 is a diagram illustrating another example of an operation of a wearable device according to an embodiment.

Referring to FIG. 23, in operation 2310, the processor 410 may identify fifth information on a trend in which a user's body temperature changes within the second cycle.

According to an embodiment, the processor 410 may identify fourth body temperature values that satisfy a designated condition according to the first cycle. For example, the processor 410 may identify a body temperature value that is the minimum among the body temperature values identified in the first cycle as the fourth body temperature value. The processor 410 may identify the fourth body temperature values by identifying the fourth body temperature value for each first cycle while the first cycle is repeated. The processor 410 may obtain fourth information on a trend in which a user's body temperature changes based on the fourth body temperature values.

The processor 410 may identify fifth information on a trend in which a user's body temperature changes in the second cycle stored in the memory 440. For example, the fifth information may be obtained through a second cycle before the fourth information is obtained, and stored in the memory 440. For example, the trend according to the fifth information may mean a trend patterned as the second cycle is repeated.

According to an embodiment, a trend in which a user's body temperature changes according to the fifth information may be referred to as a first trend. A trend in which a user's body temperature changes according to the fourth information may be referred to as a second trend.

In operation 2320, the processor 410 may identify that the shape of the first trend according to the fifth information is distinguished from the shape of the second trend according to the fourth information. For example, the first trend and the second trend may be configured in the form of a graph indicating a daily user's body temperature value.

For example, the processor 410 may identify that the shape of the first trend according to the fifth information is maintained within a range designated for each day. The processor 410 may identify that the shape of the second trend according to the fourth information is not maintained within a designated range. For example, the shape of the second trend according to the fourth information may be out of a designated range in some sections. According to the second trend, a difference between the minimum body temperature value and the maximum body temperature value in the second cycle may be identified to be equal to or greater than a designated value (e.g., 0.5 degrees to 1 degree). The processor 410 may identify that the difference between the minimum body temperature value and the maximum body temperature value in the second cycle is equal to or greater than a designated value (e.g., 0.5 degrees to 1 degree) based on the second trend according to the fourth information.

For example, the processor 410 may identify that the trend in which the body temperature of the user changes, has been changed from the second trend to the first trend. The processor 410 may identify that menarche is about to begin based on identifying that the trend in which the user's body temperature changes has changed from the second trend to the first trend. For example, the processor 410 may identify that the trend in which the user's body temperature changes has changed, from the second trend configured to maintain the body temperature within a designated range, to the first trend in which the difference between the minimum and maximum body temperature values is greater than or equal to a designated value (e.g., 0.5 degrees to 1 degree). The processor 410 may identify that ovulation has begun and menarche will be begun.

In operation 2330, the processor 410 may start identifying the user's state as one of the designated states. For example, the processor 410 may start to identify the user's state as one of the designated states based on identifying that the shape of the first trend is distinguished from the shape of the second trend.

For example, the processor 410 may identify that ovulation has begun based on identifying that the shape of the first trend is distinguished from the shape of the second trend. The processor 410 may provide a women's health service based on identifying that ovulation has begun. As an example, the processor 410 may start to identify the user's state as one of the designated states based on identifying that ovulation has begun. The processor 410 may start to identify the user's state as one of menstrual phase status, follicular phase status, ovulation phase status, and luteal phase status based on identifying that ovulation has begun.

Referring to FIG. 24, the processor 410 may provide a notification indicating that the user's state has begun to be identified as one of the designated states on the screen 2410. The processor 410 may provide a notification indicating that the user's menarche has begun on the screen 2410.

According to an embodiment, the processor 410 may display a text 2411 indicating that the user's menarche has begun on the screen 2410. The processor 410 may display text 2412 about the user's expected menarche date together.

According to an embodiment, the processor 410 may display a visual object for providing various information to the user together with the text 2411 and the text 2412. For example, the processor 410 may display a visual object 2430 for providing information about menarche to the user. For example, the processor 410 may perform a connection to a designated uniform resource locator (URL) based on a user input for the visual object 2430. As another example, the processor 410 may display visual data (e.g., text, picture, or image) related to women's health on the display 420 based on a user input to the visual object 2430.

According to an embodiment, the processor 410 may display a visual object 2440 for performing a designated activity together with the text 2411 and the text 2412. For example, the processor 410 may display a visual object 2440 for the user to make a phone call to a parent or a counseling center. The processor 410 may perform a designated activity based on a user input for the visual object 2440. According to an embodiment, similar to the visual object 2230 of FIG. 22, the processor 410 may transmit a signal for performing a designated activity to an external device based on a user input for the visual object 2440.

According to the above-described embodiments, the processor 410 may identify the user's body temperature values for one day (or for 24 hours). The processor 410 may identify a circadian rhythm based on the user's body temperature values identified during the day, and pattern a circadian rhythm. The processor 410 may identify a trend in which the user's body temperature (i.e., basal body temperature) is changed for one month (or menstrual cycle) based on the patterned circadian rhythm. The processor 410 may identify an individual body temperature change trend by patterning a trend in which a user's body temperature changes for one month. The processor 410 may provide women's health services based on individual body temperature change trends.

According to an embodiment, a wearable device(e.g., the wearable device 400) may comprise a non-contact temperature sensor (e.g., temperature sensors 502), a biometric sensor (e.g., PPG sensors 501), a motion sensor (e.g., motion sensors 503), a memory (e.g., memory 440), and an at least one processor(e.g., processor 410) operably coupled to the temperature sensor, the biometric sensor, the motion sensor, and the memory, configured to while identifying that the user is in a stable state within a first cycle, obtain, using the temperature sensor, first body temperature data of the user according to a first cycle; while identifying that the user is in an unstable state within the first cycle, obtain, using the motion sensor, values for indicating a change of motion of the user according to a second cycle distinct from the first cycle; in response to identifying that each of the values for indicating the change of motion of the user is less than a reference value, obtain second body temperature data through the temperature sensor; obtain, based on the first body temperature data and the second body temperature data, first information on a trend in which body temperature of the user changes within the first cycle; and provide a service based at least part on the first information.

According to an embodiment, the biometric sensor may include photoplethysmography (PPG) sensor; the stable state may include a sleep state; the unstable state may include a non-sleep state; and wherein the at least one processor may be configured to obtain, while identifying, using the PPG sensor, that the user is in the sleep state within the first cycle, using the temperature sensor, the first body temperature data of the user according to the first cycle; and while identifying, using the PPG sensor, that the user is in the non-sleep state within the first cycle, obtain the values for indicating the change of motion of the user using the motion sensor according to a second period different from the first period.

According to an embodiment, the first information on the trend may include first body temperature values obtained at a plurality of time points within the first cycle; and the at least one processor may be further configured to identify second information on a trend in which the body temperature of the user changes within the first cycle, stored in the memory; identify, based on the second information, second body temperature values mapped to the plurality of time points; obtain, based on a difference between the first body temperature values and the second body temperature values mapped to the plurality of time points, third body temperature values mapped to the plurality of time points, by changing at least one of the first body temperature values; obtain, based on the third body temperature values, third information on a trend in which the body temperature of the user changes within the first cycle; and identify, based on the second information and the third information, a state of the user from among designated states circulated along the second cycle exceeding the first cycle.

According to an embodiment, the at least one processor may be further configured to identify, based on the second information and the third information, fourth body temperature values that satisfy a designated condition according to the first cycle; obtain, based on the fourth body temperature values, fourth information on a trend in which the body temperature of the user changes within the second cycle exceeding the first cycle, and identify, based on the fourth information, the state of the user from among the designated states circulated along the second cycle.

According to an embodiment, a standard deviation value of the third body temperature values with respect to the second body temperature values may be less than a standard deviation value of the first body temperature values with respect to the second body temperature values.

According to an embodiment, the at least one processor may be further configured to update, based on the second information and the third information, the trend in which the body temperature of the user changes within the first cycle according to the second information.

According to an embodiment, the at least one processor may be configured to identify, based on the second information, a critical range for the body temperature of the user, and identify at least one of the first body temperature values outside the identified critical range.

According to an embodiment, the designated states may include menstrual status, follicular status, ovulatory status, and luteal phase status.

According to an embodiment, the wearable device may further comprise a display, and the at least one processor may be further configured to identify, based on the fourth information, a time point in which an event related to the user will be occurred, and display, using the display, a guide for the time point in which the event related to the user will be occurred.

According to an embodiment, the at least one processor may be further configured to identify, based on the fourth information, an abnormal symptom of the user, and provide, based on the identified abnormal symptom of the user, a notification for the identified abnormal symptom of the user.

According to an embodiment, the wearable device may further comprise a display, and the at least one processor may be further configured to display a visual object for performing a designated activity with the notification, and transmit, based on identifying the input to the visual object, a signal for performing the designated activity to an external device connected to the wearable device.

According to an embodiment, the at least one processor may be further configured to identify fifth information on a trend in which the body temperature of the user changes within the 28 day - one month cycle, stored in the memory, identify that a shape of the first trend according to the fifth information is distinct from a shape of the second trend according to the fourth information, and start identifying the state of the user as one of the designated states, based on identifying that the shape of the first trend is distinct from the shape of the second trend.

According to an embodiment, the at least one processor may be further configured to identify a user input during identifying, using the PPG sensor, that the user is within a non-sleep state within the first cycle, and obtain, based on the user input, third body temperature data using the temperature sensor.

According to an embodiment, a method of a wearable device (e.g., a wearable device 400) may comprise obtaining, using a temperature sensor(e.g., a temperature sensor 502), while identifying that a user is in a stable state within a first cycle, first body temperature data of the user according to a first cycle; while identifying that the user is in an unstable state within the first cycle, obtaining, using the motion sensor(e.g., motion sensor 503), values for indicating a change of motion of the user according to a second cycle distinct from the first cycle; in response to identifying that each of the values for indicating the change of motion of the user is less than a reference value, obtaining second body temperature data through the temperature sensor; obtaining, based on the first body temperature data and the second body temperature data, first information on a trend in which body temperature of the user changes within the first cycle; and providing a service based at least part on the first information.

According to an embodiment, the biometric sensor may include photoplethysmography (PPG) sensor, the stable state may include a sleep state, the unstable state may include a non-sleep state, and the method may further comprise obtaining, while identifying, using the PPG sensor, that the user is in the sleep state within the first cycle, using the temperature sensor, the first body temperature data of the user according to the first cycle, and while identifying, using the PPG sensor, that the user is in the non-sleep state within the first cycle, obtaining the values for indicating the change of motion of the user using the motion sensor according to the second cycle distinct from the first cycle.

According to an embodiment, the first information on the trend may include first body temperature values obtained at a plurality of time points within the first cycle, and the method may further comprise identifying second information on a trend in which the body temperature of the user changes within the first cycle, stored in a memory, identifying, based on the second information, second body temperature values mapped to the plurality of time points; obtaining, based on a difference between the first body temperature values and the second body temperature values mapped to the plurality of time points, third body temperature values mapped to the plurality of time points, by changing at least one of the first body temperature values; obtaining, based on the third body temperature values, third information on a trend in which the body temperature of the user changes within the first cycle; and identifying, based on the second information and the third information, a state of the user from among designated states circulated along the second cycle exceeding the first cycle.

According to an embodiment, the method may further comprise identifying, based on the second information and the third information, fourth body temperature values that satisfy a designated condition according to the first cycle; obtaining, based on the fourth body temperature values, fourth information on a trend in which the body temperature of the user changes within the second cycle exceeding the first cycle; and identifying, based on the fourth information, the state of the user from among the designated states circulated along the second cycle.

According to an embodiment, a standard deviation value of the third body temperature values with respect to the second body temperature values may be less than a standard deviation value of the first body temperature values with respect to the second body temperature values.

According to an embodiment, the method may further comprise updating, based on the second information and the third information, the trend in which the body temperature of the user changes within the first cycle according to the second information.

According to an embodiment, the method may further comprise an operation of identifying a time point at which the event related to the user will be occurred based on the fourth information, and an operation of displaying a guide for a time point at which the event related to the user will be occurred by using the display.

According to an embodiment, the method may further include identifying, based on the fourth information, an abnormal symptom of the user, and providing a notification of the abnormal symptom of the identified user based on the identified abnormal symptom of the user.

According to an embodiment, a non-transitory computer readable storage medium may store one or more programs, the one or more programs including instructions, which, when being executed by at least one processor (e.g., the processor 410) of a wearable device with a temperature sensor(e.g., temperature sensor 502), a biometric sensor (e.g., PPG sensor 501), a motion sensor(e.g., motion sensor 503), and a memory(e.g., memory 440), cause the electronic device to obtain, while identifying that the user is in a stable state within a first cycle, using the temperature sensor, first body temperature data of the user according to a first cycle; while identifying that the user is in an unstable state within the first cycle, obtain, using the motion sensor, values for indicating a change of motion of the user according to a second cycle distinct from the first cycle; in response to identifying that each of the values for indicating the change of motion of the user is less than a reference value, obtain second body temperature data through the temperature sensor; obtain, based on the first body temperature data and the second body temperature data, first information on a trend in which body temperature of the user changes within the first cycle; and provide a service based at least part on the first information.

The electronic device according to an embodiment may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that an embodiment of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with an embodiment of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, The module may be implemented in a form of an application-specific integrated circuit (ASIC).

An embodiment as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to an embodiment of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities, and some of the multiple entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to an embodiment, the integrated component may still perform one or more functions of each of the plurality of components in the same or similar manner as they are performed by a corresponding one of the plurality of components before the integration. According to an embodiment, operations performed by the module, the program, or another component may be carried out sequentially, in parallel, repeatedly, or heuristically, or one or more of the operations may be executed in a different order or omitted, or one or more other operations may be added.

## Claims

1. A wearable device comprising:
a non-contact temperature sensor;
a biometric sensor;
a motion sensor;
a memory; and
an at least one processor operably coupled to the non-contact temperature sensor, the biometric sensor, the motion sensor, and the memory, configured to:
while identifying that a user is in a stable state within a first cycle, obtain, using the non-contact temperature sensor, first body temperature data of the user according to a first period,
while identifying that the user is in an unstable state within the first cycle, obtain, using the motion sensor, values for indicating a change of motion of the user according to a second period distinct from the first period,
in response to identifying that each of the values for indicating the change of motion of the user is less than a reference value, obtain second body temperature data through the non-contact temperature sensor,
obtain, based on the first body temperature data and the second body temperature data, first information on a trend in which body temperature of the user changes within the first cycle, and
provide a service based at least part on the first information.

2. The wearable device according to claim 1, wherein the biometric sensor includes photoplethysmography (PPG) sensor,
wherein the stable state includes a sleep state,
wherein the unstable state includes a non-sleep state, and
wherein the at least one processor is configured to:
while identifying, using the PPG sensor, that the user is in the sleep state within the first cycle, obtain, using the non-contact temperature sensor, the first body temperature data of the user according to the first cycle, and
while identifying, using the PPG sensor, that the user is in the non-sleep state within the first cycle, obtain values for indicating the change of motion of the user using the motion sensor according to a second period different from the first period.

3. The wearable device according to claim 1, wherein the first information on the trend includes first body temperature values obtained at a plurality of time points within the first cycle, and
wherein the at least one processor is further configured to:
identify second information on a trend in which the body temperature of the user changes within the first cycle, stored in the memory,
identify, based on the second information, second body temperature values mapped to the plurality of time points,
obtain, based on a difference between the first body temperature values and the second body temperature values mapped to the plurality of time points, third body temperature values mapped to the plurality of time points, by changing at least one of the first body temperature values,
obtain, based on the third body temperature values, third information on a trend in which the body temperature of the user changes within the first cycle, and
identify, based on the second information and the third information, a state of the user from among designated states circulated along the second cycle exceeding the first cycle.

4. The wearable device according to claim 3, wherein the at least one processor is further configured to:
identify, based on the second information and the third information, fourth body temperature values that satisfy a designated condition according to the first cycle,
obtain, based on the fourth body temperature values, fourth information on a trend in which the body temperature of the user changes within the second cycle exceeding the first cycle, and
identify, based on the fourth information, the state of the user from among the designated states circulated along the second cycle.

5. The wearable device according to claim 3, wherein a standard deviation value of the third body temperature values with respect to the second body temperature values is less than a standard deviation value of the first body temperature values with respect to the second body temperature values.

6. The wearable device according to claim 3, wherein the at least one processor is further configured to:
update, based on the second information and the third information, the trend in which the body temperature of the user changes within the first cycle according to the second information.

7. The wearable device according to claim 3, wherein the at least one processor is configured to:
identify, based on the second information, a critical range for the body temperature of the user, and
identify at least one of the first body temperature values outside the identified critical range.

8. The wearable device according to claim 4, wherein the designated states include menstrual status, follicular status, ovulatory status, and luteal phase status.

9. The wearable device according to claim 4, further comprising a display, and wherein the at least one processor is further configured to:
identify, based on the fourth information, a time point in which an event related to the user will be occur, and
display, using the display, a guide for the time point in which the event related to the user will be occurred.

10. The wearable device according to claim 4, wherein the at least one processor is further configured to:
identify, based on the fourth information, an abnormal symptom of the user, and
provide, based on the identified abnormal symptom of the user, a notification for the identified abnormal symptom of the user.

11. The wearable device according to claim 10, further comprising a display, and wherein the at least one processor is further configured to:
display a visual object for performing a designated activity with the notification, and
transmit, based on identifying an input to the visual object, a signal for performing the designated activity to an external device connected to the wearable device.

12. The wearable device according to claim 4, wherein the at least one processor is further configured to:
identify fifth information on a trend in which the body temperature of the user changes within the second cycle, stored in the memory,
identify that a shape of a first trend according to the fifth information is distinct from a shape of a second trend according to the fourth information, and
start identifying the state of the user as one of the designated states, based on identifying that the shape of the first trend is distinct from the shape of the second trend.

13. The wearable device according to claim 2, wherein the at least one processor is further configured to:
identify a user input during identifying, using the PPG sensor, that the user is within a non-sleep state within the first cycle, and
obtain, based on the user input, third body temperature data using the non-contact temperature sensor.

14. A method of a wearable device comprising:
while identifying that a user is in a stable state within a first cycle, obtaining, using a non-contact temperature sensor, first body temperature data of the user according to a first period,
while identifying that the user is in an unstable state within the first cycle, obtaining, using a motion sensor, values for indicating a change of motion of the user according to a second period distinct from the first period,
in response to identifying that each of the values for indicating the change of motion of the user is less than a reference value, obtaining second body temperature data through the non-contact temperature sensor,
obtaining, based on the first body temperature data and the second body temperature data, first information on a trend in which body temperature of the user changes within the first cycle, and
providing a service based at least part on the first information.

15. A non-transitory computer readable storage medium storing one or more programs, the one or more programs including instructions, which, when being executed by at least one processor of a wearable device with a non-contact temperature sensor, a biometric sensor, a motion sensor, and a memory, cause the electronic device to:
while identifying that a user is in a stable state within a first cycle, obtain, using the non-contact temperature sensor, first body temperature data of the user according to a first period,
while identifying that the user is in an unstable state within the first cycle, obtain, using the motion sensor, values for indicating a change of motion of the user according to a second period distinct from the first period,
in response to identifying that each of the values for indicating the change of motion of the user is less than a reference value, obtain second body temperature data through the non-contact temperature sensor,
obtain, based on the first body temperature data and the second body temperature data, first information on a trend in which body temperature of the user changes within the first cycle, and
provide a service based at least part on the first information.
